# EUROPEAN PATENT APPLICATION

(11) **EP 4 796 173 A1**
(43) Date of publication of application: **26.08.2026**
(21) Application number: 26158086.4
(22) Date of filing: 12.02.2026
(51) Int. Cl.: A61P 7/02, C07C 323/12, C07F 9/50, A61K 31/095, A61K 31/66, G01N 33/86

(54) **THROMBOLYTIC ACTIVITY OF DISULFIDE REDUCING AGENTS**

(30) Priority: 12.02.2025 US 202563757535 P
(71) Applicant: Georgia Tech Research Corporation, Atlanta, GA 30318 (US)
(72) Inventor: KU, David N., Atlanta, GA 30318 (US); BRESETTE, Christopher, Atlanta, GA 30318 (US); MACHENAUD, Edouard, Atlanta, GA 30318 (US)
(74) Representative: Snodin, Michael D.

(57) **Abstract**

The present disclosure relates to a method of treating a disease or disorder associated with thrombus formation in a subject in need thereof, comprising administering to the subject a therapeutically effective amount of a compound represented by structural formula (I) or structural formula (II) or a pharmaceutically acceptable salt thereof:

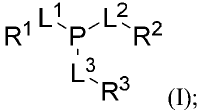

HO-M-SH (II).

## Description

### RELATED APPLICATION

This application claims the benefit of priority to United States Provisional Patent Application serial number 63/757,535, filed February 12, 2025.

### BACKGROUND OF THE INVENTION

The diversity of thrombi composition reinforces the need for thrombolytics to treat mortal conditions such as myocardial infarction or ischemic stroke. Current FDA-approved thrombolytics are based on the tissue plasminogen activator (tPA) mechanism that exclusively targets fibrin. Thrombi formed under arterial high shear rate conditions are often fibrin-deficient and enriched in von Willebrand Factor (VWF) fibers, which polymerize through disulfide bonding. This structural difference likely explains the limited efficacy of current fibrinolytic therapies. Accordingly, efficient agents targeting von Willebrand Factor fibers are needed.

### SUMMARY OF THE INVENTION

The present disclosure relates to a method of treating a disease or disorder associated with thrombus formation in a subject in need thereof, comprising administering to the subject a therapeutically effective amount of a compound represented by structural formula (I) or structural formula (II) or a pharmaceutically acceptable salt thereof:

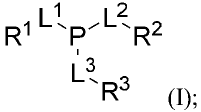

HO^{-M-SH} (II),

wherein
each L¹, L², and L³ is independently C₁₋₆ alkylene;
M is C₂₋₆ alkylene;
each R¹, R², and R³ is independently selected from C(O)OR⁵, OR⁴, C(O)NR⁶R⁷, NR⁸C(O)R⁹, and NR¹⁰R¹¹,
each R⁴, R⁵, R⁶, R⁷, R⁸, R⁹, R¹⁰, and R¹¹ is selected from H and C₁₋₆ alkyl, or
R⁶ and R⁷, or R¹⁰ and R¹¹, together with the nitrogen atom to which they are attached form a 3- to 8-membered heterocyclyl,
wherein each C₁₋₆ alkylene, C₂₋₆ alkylene, and C₁₋₆ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
wherein
each R¹¹, R¹², R¹³, R¹⁴, R^{14*}, R¹⁵, R^{15*}, R¹⁶, R¹⁷, R^{16*}, R^{17*}, R¹⁸, R^{18a}, R¹⁹, R²⁰, R^{20a}, R^{20b}, R²¹, R²², R^{21*}, R^{22*}, R²³, R²⁴, R²⁵, R²⁶, and R²⁷ is independently selected from H, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl, or
one or more of the pairs of variables selected from R¹² and R¹³, R¹⁶ and R¹⁷, R^{16*} and R^{17*}, R²¹ and R²², R^{21*} and R^{22*}, and R²⁵ and R²⁶, together with the nitrogen to which they are attached, form a 3-8 membered ring, and
each of the substituents selected from C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl is further optionally substituted with 1 to 3 substituents independently selected from deuterium, oxo, F, Cl, Br, CN, NO₂, OR^{14*}, SR^{15*}, NR^{16*}R^{17*}, C₁₋₆ alkyl, C₁₋₆ haloalkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.

The present disclosure relates to a method of treating thrombus formation in a cavity or device, comprising contacting the cavity or device with a compound represented by structural formula (I) or structural formula (II) or a pharmaceutically acceptable salt thereof:

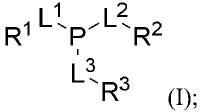

**HO-M-SH** (II),

wherein
each L¹, L², and L³ is independently C₁₋₆ alkylene;
M is C₂₋₆ alkylene;
each R¹, R², and R³ is independently selected from C(O)OR⁵, OR⁴, C(O)NR⁶R⁷, NR⁸C(O)R⁹, and NR¹⁰R¹¹,
each R⁴, R⁵, R⁶, R⁷, R⁸, R⁹, R¹⁰, and R¹¹ is selected from H and C₁₋₆ alkyl, or
R⁶ and R⁷, or R¹⁰ and R¹¹, together with the nitrogen atom to which they are attached form a 3- to 8-membered heterocyclyl,
wherein each C₁₋₆ alkylene, C₂₋₆ alkylene, and C₁₋₆ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
wherein
each R¹¹, R¹², R¹³, R¹⁴, R^{14*}, R¹⁵, R^{15*}, R¹⁶, R¹⁷, R^{16*}, R^{17*}, R¹⁸, R^{18a}, R¹⁹, R²⁰, R^{20a}, R^{20b}, R²¹, R²², R^{21*}, R^{22*}, R²³, R²⁴, R²⁵, R²⁶, and R²⁷ is independently selected from H, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl, or
one or more of the pairs of variables selected from R¹² and R¹³, R¹⁶ and R¹⁷, R^{16*} and R^{17*}, R²¹ and R²², R^{21*} and R^{22*}, and R²⁵ and R²⁶, together with the nitrogen to which they are attached, form a 3-8 membered ring, and
each of the substituents selected from C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl is further optionally substituted with 1 to 3 substituents independently selected from deuterium, oxo, F, Cl, Br, CN, NO₂, OR^{14*}, SR^{15*}, NR^{16*}R^{17*}, C₁₋₆ alkyl, C₁₋₆ haloalkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.

### BRIEF DESCRIPTION OF THE DRAWINGS

Fig. 1 is a schematic representation of a microfluidic device with trifurcation for formation and lysis of high shear thrombi showing the lysis experiment timeline.
Fig. 2 is a plot demonstrating flow rate curves obtained with *in vitro* model in the microfluidic device in Fig. 1. The 0 minute time point corresponds to lysis onset. Lysis efficacy of tris(2-carboxyethyl)phosphine (TCEP) at 100 µM and 0.1 µM is shown by increase of flow rate versus PBS control. The horizontal shift of the TCEP 0.1 µM curve is due to variability in time to reach occlusion.

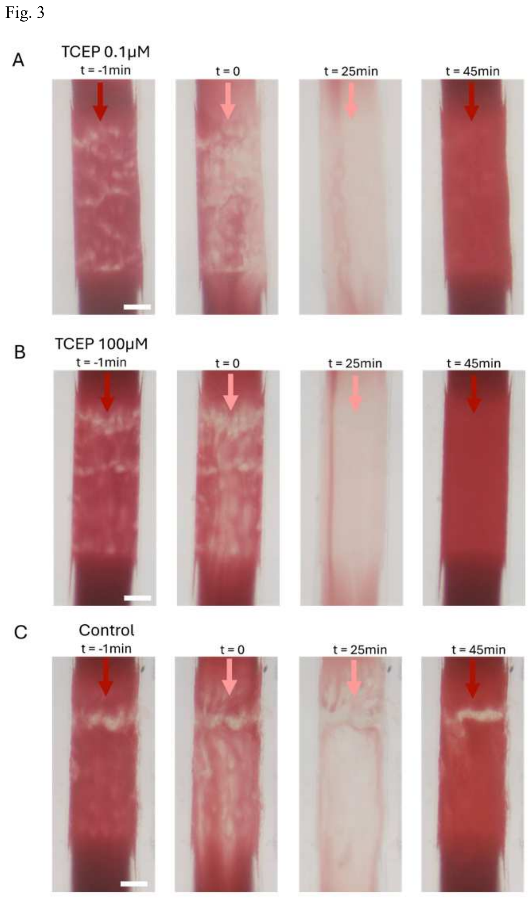

Fig. 3 shows a series of light microscopy images before and after perfusion of TCEP and PBS as a vehicle through high shear thrombi. (A) Perfusion of TCEP 0.1 µM. Thicker part of thrombus forms in the upstream part of the highest shear region of the microfluidic. At t=0, the TCEP lytic solution with PBS as vehicle is perfused, and flow appears more transparent than the darker, red whole blood. At t=25 min, the flow diverged around three islets. Almost transparent flow does not allow for visual evaluation of thrombus lysis. Thus after 45 minutes, whole blood is flowing again, and white spots can be observed. (B) Perfusion of TCEP 100 µM. After 45 minutes, no thrombus material remains. (C) Perfusion of PBS only. The thrombus is still present after 45 minutes.
Fig. 4 is a plot of flow rate versus time at varying concentration of TCEP and PBS as control (TCEP: 10 mM, 1 mM, and 0.1 mM). The plot demonstrates the efficacy of TCEP at lysing high shear thrombi at millimolar concentration levels.
Fig. 5 is a plot demonstrating efficacy of 2-mercaptoethanol (BME) at lysing high shear thrombi at millimolar concentration levels. Lysis flow rate curves are shown at concentrations 10 mM, 1 mM, and 0.1 mM, compared to PBS control.
Fig. 6 is a plot demonstrating efficacy of BME at lysing high shear thrombi at millimolar concentration levels. Lysis flow rate curves are shown at a concentration of 10 mM (panel A), and at 1 mM (panel B).
Fig. 7 is a plot of three lysis flow rate curves versus time showing the lysis efficacy of tris(hydroxypropyl)phosphine (THPP) at a 5 mM concentration level on high shear thrombi.
Fig. 8 is a plot showing a dose response curve of TCEP efficacy. Four parameters logistic regression (4PL) vs TCEP dose in µM, R²=0.85. 4PL model parameters: Min Efficacy=4.6; Max Efficacy=80.6; EC50=0.50; Hill Slope=0.43.
Fig. 9 is a plot of three lysis flow rate curves versus time where the vehicle for TCEP is whole human blood and not PBS. The 0 minute time point corresponds to lysis onset. This plot demonstrates the efficacy of TCEP at lysing high shear thrombi even when blood is the vehicle.

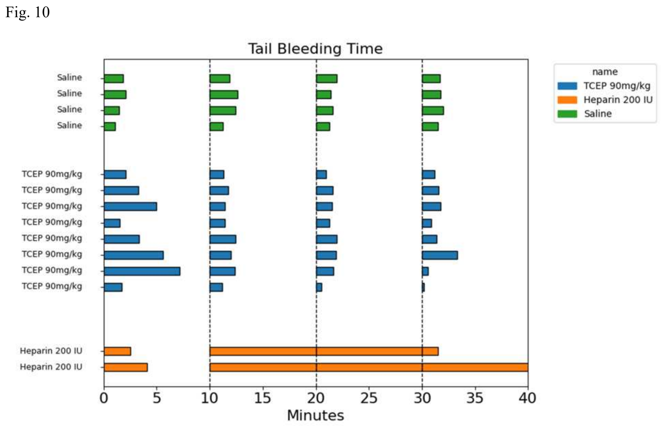

Fig. 10 demonstrates *in vivo* bleeding safety of TCEP in a murine tail vein transection model. Bleeding times were measured in fully anesthetized C57BL/6 mice following standardized lateral tail vein transection and subsequent mechanical clot challenges at 10, 20, and 30 min. Initial bleeding reflects primary hemostasis, whereas rebleeding after challenge reflects secondary hemostasis. TCEP 90 mg/kg modestly prolonged initial bleeding relative to saline but did not prevent occlusion and did not increase bleeding following clot challenges, in contrast to heparin, which markedly prolonged rebleeding times.

### DETAILED DESCRIPTION OF THE INVENTION

Acute ischemic stroke (AIS) results from the sudden occlusion of cerebral arteries blocking the critical supply of blood to brain tissue. In the United States, 17.5% of death from cardiovascular disease are due to stroke, 87% of strokes are ischemic, and stroke-related costs were nearly US$56.2 billion between 2019 and 2020. Therefore, the search for a safe, efficacious, and fast-acting treatment remains a priority as timely recanalization is decisive to minimizing neuronal damage and preventing death.

Recombinant tissue plasminogen activator (r-tPA) is the current gold standard and only FDA-approved pharmacological treatment of AIS. r-tPA can effectively dissolve fibrin-rich thrombi. However, its use is limited by a present severe risk of hemorrhagic complications and a short 4.5 hours treatment window from stroke symptoms onset. Clinical studies found a limited recanalization rate of 30% using r-tPA and no statistically significant improvement in overall mortality. Increasing clinical evidence indicates that thrombi are composed not only of fibrin, platelets, and red blood cells but also include components such as von Willebrand factor (VWF) and neutrophil extracellular traps (NETs). These additional structural elements likely contribute to the reduced efficacy of r-tPA and highlight its limitations as a universal thrombolytic agent.

Occlusive arterial thrombi responsible for ischemic stroke are either cardioembolic clots, cryptogenic clots, or thrombi formed *in situ.* Thromboembolic clots are typically fibrin-rich and often originate from the heart in patients with atrial fibrillation. In contrast, *in situ* thrombi are formed following atherosclerotic plaque rupture and under high shear hemodynamic conditions. High shear forces inhibit fibrinogen adhesion but instead promote the elongation of VWF and their aggregation with platelets to form VWF-rich thrombotic plugs.

Recent findings have demonstrated that r-tPA has limited efficacy in lysing such high-shear thrombi. tPA functions as a serine protease, catalyzing the conversion of plasminogen to plasmin, which subsequently cleaves fibrin to facilitate thrombus lysis. However, its activity appears nil against VWF, which predominantly comprise disulfide bonds resistant to fibrinolysis. Targeting disulfide bonds lyses high shear thrombi, addressing a critical gap in the treatment of ischemic stroke.

In some embodiments, the present disclosure relates to examination of the efficacy of disulfide bond reducing agents using a 3D printed microfluidic chip allowing for the formation of high shear thrombi and the rapid delivery of thrombolytic agents. Light microscopy and mass balance data shows that the perfusion of tris(2-carboxyethyl)phosphine (TCEP), tris(hydroxypropyl)phosphine (THPP), and 2-mercaptoethanol (BME) can lyse platelet-VWF thrombi within minutes.

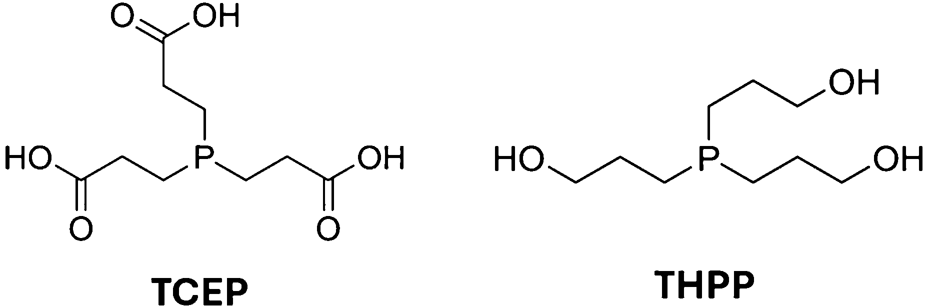

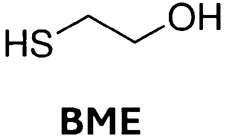

The disulfide bond reducing agents TCEP, THPP, and BME proved efficacious in lysing high shear thrombi while control using PBS did not lyse thrombi. High shear thrombi formation has been shown to rely primarily on Shear-Induced Platelet Aggregation (SIPA). High shear thrombi have been shown to be rich in platelets and VWF while poor in fibrin and RBCs. VWF is composed of repeating monomeric subunits of molecular weight ~250kDa that can polymerize into ultra long fibers exceeding 10,000kDa. Disulfide bonds hold together the multimers at both the N-terminus and C-terminus, and the use of S-S reducing agents on stretched VWF via molecular combing fragmented the multimer into protomers. Thiol exchange resulting in disulfide bond formation has also been shown to be an important process in the adhesion of VWF to platelets. More recently, VWF has been shown to be the main structural fiber holding platelets together inducing occlusion. The unexpected efficacious lytic power of TCEP, THPP, and BME on thrombi likely results from their disulfide bond reduction potential, allowing for a new way to treat arterial thrombosis involving clot containing VWF. Experimental results are described below.

### Definitions

In this specification and in the claims that follow, reference will be made to many terms, which shall be defined to have the following meanings: Throughout the present specification, numerical ranges are provided for certain quantities. It is to be understood that these ranges comprise all values and subranges therein. Thus, e.g., the range "from 1 to 10" includes all possible values therein (e.g., 1, 2, 3, 4, 5, 6, 7, 8, 9, and 10) and all possible ranges therein (e.g., 1-9, 2-8, 3-7, 4-6, 1-8, 2-7, 3-6, 4-5, 1- 7, 2-6, 3-5, 1-6, 2-5, 3-4, etc.). Furthermore, all values within a given range may be an endpoint for the range encompassed thereby (e.g., the range 1-10 includes the ranges with endpoints such as 5-10, 6-10, etc.).

As used in the description and the appended claims, the singular forms "a," "an," and "the" include plural referents unless the context dictates otherwise. Thus, for example, reference to "a composition" includes mixtures of two or more such compositions, reference to "an inhibitor" includes mixtures of two or more such inhibitors and the like.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various stereoisomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high-performance liquid chromatography (HPLC) and the formation and crystallization of chiral salts; or preferred isomers can be prepared by asymmetric syntheses. See, for example, Jacques et al., Enantiomers, Racemates and Resolutions, Wiley Interscience, New York, 1981; Wilen et al., Tetrahedron 33:2725 (1977); Eliel, E.L. Stereochemistry of Carbon Compounds, McGraw-Hill, NY, 1962; and Wilen, S.H., Tables of Resolving Agents and Optical Resolutions p. 268, E.L. Eliel, Ed., Univ. of Notre Dame Press, Notre Dame, IN 1972. The invention additionally encompasses compounds as individual isomers substantially free of other isomers, and alternatively, as mixtures of various isomers.

In a formula, --- is absent or a single bond, and -̅ -̅ -̅ -̅ or -̅ -̅ -̅ -̅ is a single or double bond. An asterisk (*) next to an atom indicates that the atom is a stereocenter of unknown absolute configuration. For example, in a pair of enantiomers each can be depicted by a chemical structure with an asterisk (*) next to the stereocenter, which would indicate that the absolute configuration for the stereocenter of a given enantiomer is not defined.

In a formula, 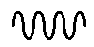 indicates a point of attachment of the moiety to the remaining part of the molecule.

Unless otherwise stated, structures depicted herein are also meant to include all isomeric (e.g., enantiomeric, diastereomeric, and geometric (or conformational)) forms of the structure; for example, the R and S configurations for each stereocenter. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention.

Unless otherwise stated, structures depicted herein are also meant to include compounds that differ only in the presence of one or more isotopically enriched atoms. For example, compounds having the present structures except for the replacement of hydrogen by deuterium or tritium, replacement of ¹⁹F with ¹⁸F, or the replacement of ¹²C with ¹³C or ¹⁴C are within the scope of the disclosure. Such compounds are useful, for example, as analytical tools or probes in biological assays.

When a range of values is listed, it is intended to encompass each value and sub-range within the range. For example, "C₁₋₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The term "aliphatic" refers to alkyl, alkenyl, alkynyl, and carbocyclic groups. Likewise, the term "heteroaliphatic" refers to heteroalkyl, heteroalkenyl, heteroalkynyl, and heterocyclic groups.

The term "alkyl" refers to a radical of a straight-chain or branched saturated hydrocarbon group having from 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), propyl (C₃) *(e.g.,* n-propyl, isopropyl), butyl (C₄) *(e.g.,* n-butyl, tert-butyl, sec-butyl, iso-butyl), pentyl (C₅) *(e.g.,* n-pentyl, 3-pentanyl, amyl, neopentyl, 3- methyl-2-butanyl, tertiary amyl), and hexyl (C₆) *(e.g.,* n-hexyl). Additional examples of alkyl groups include n-heptyl (C₇), n-octyl (Cs), and the like. Unless otherwise specified, each instance of an alkyl group is independently unsubstituted (an "unsubstituted alkyl") or substituted (a "substituted alkyl") with one or more substituents (e.g., halogen, such as F). In certain embodiments, the alkyl group is an unsubstituted C₁₋₁₀ alkyl (such as unsubstituted C₁₋₆ alkyl, *e.g.,* -CH₃ (Me), unsubstituted ethyl (Et), unsubstituted propyl (Pr, *e.g.,* unsubstituted n-propyl (n-Pr), unsubstituted isopropyl (i-Pr)), unsubstituted butyl (Bu, e.g., unsubstituted n-butyl (n-Bu), unsubstituted tert-butyl (tert-Bu or t-Bu), unsubstituted sec-butyl (sec-Bu), unsubstituted isobutyl (i-Bu)). In certain embodiments, the alkyl group is a substituted C₁₋₁₀ alkyl (such as substituted C₁₋₆ alkyl, *e.g.,* -CF₃, Bn).

The term "haloalkyl" refers to a substituted alkyl group, wherein one or more of the hydrogen atoms are independently replaced by a halogen, e.g., fluoro, bromo, chloro, or iodo. In some embodiments, the haloalkyl moiety has 1 to 8 carbon atoms ("C₁₋₈ haloalkyl"). In some embodiments, the haloalkyl moiety has 1 to 6 carbon atoms ("C₁₋₆ haloalkyl"). In some embodiments, the haloalkyl moiety has 1 to 4 carbon atoms ("C₁₋₄ haloalkyl"). In some embodiments, the haloalkyl moiety has 1 to 3 carbon atoms ("C₁₋₃ haloalkyl"). In some embodiments, the haloalkyl moiety has 1 to 2 carbon atoms ("C₁₋₂ haloalkyl"). Examples of haloalkyl groups include -CHF₂, -CH₂F, -CF₃, -CH₂CF₃, -CF₂CF₃, -CF₂CF₂CF₃, -CCl₃, -CFCl₂, - CF₂Cl, and the like.

The term "deuteroalkyl" refers to an alkyl group, wherein one or more of the hydrogen atoms are independently replaced by deuterium. In some embodiments, the deuteroalkyl moiety has 1 to 8 carbon atoms ("C₁₋₈ deuteroalkyl"). In some embodiments, the deuteroalkyl moiety has 1 to 6 carbon atoms ("C₁₋₆ deuteroalkyl"). In some embodiments, the deuteroalkyl moiety has 1 to 4 carbon atoms ("C₁₋₄ deuteroalkyl "). In some embodiments, the deuteroalkyl moiety has 1 to 3 carbon atoms ("C₁₋₃ deuteroalkyl "). In some embodiments, the deuteroalkyl moiety has 1 to 2 carbon atoms ("C₁₋₂ deuteroalkyl"). In some embodiments, the deuteroalkyl moiety is C₁, C₂, C₃, C₄, C₅, or C₆ deuteroalkyl. A deuteroalkyl moiety having n carbon atoms can have from 1 to 2n+1 deuterium atoms. Examples of deuteroalkyl groups include -CHD₂, -CH₂D, -CD₃, -CH₂CD₃, -CD₂CD₃, -CD₂CD₂CD₃, -CH(CD₃)₂, -CD(CD₃)₂, - C(CD₃)₃, and the like.

The term "hydroxyalkyl" is a substituted alkyl group, wherein one or more of the hydrogen atoms are independently replaced by a hydroxyl. In some embodiments, the hydroxyalkyl moiety has 1 to 8 carbon atoms ("C₁₋₈ hydroxyalkyl"). In some embodiments, the hydroxyalkyl moiety has 1 to 6 carbon atoms ("C₁₋₆ hydroxyalkyl"). In some embodiments, the hydroxyalkyl moiety has 1 to 4 carbon atoms ("C₁₋₄ hydroxyalkyl"). In some embodiments, the hydroxyalkyl moiety has 1 to 3 carbon atoms ("C₁₋₃ hydroxyalkyl"). In some embodiments, the hydroxyalkyl moiety has 1 to 2 carbon atoms ("C₁₋₂ hydroxyalkyl").

The term "alkoxy" refers to an alkyl group, as defined herein, appended to the parent molecular moiety through an oxygen atom. In some embodiments, the alkoxy moiety has 1 to 8 carbon atoms ("C₁₋₈ alkoxy"). In some embodiments, the alkoxy moiety has 1 to 6 carbon atoms ("C₁₋₆ alkoxy"). In some embodiments, the alkoxy moiety has 1 to 4 carbon atoms ("C₁₋₄ alkoxy"). In some embodiments, the alkoxy moiety has 1 to 3 carbon atoms ("C₁₋₃ alkoxy"). In some embodiments, the alkoxy moiety has 1 to 2 carbon atoms ("C₁₋₂ alkoxy"). Representative examples of alkoxy include, but are not limited to, methoxy, ethoxy, propoxy, 2-propoxy, butoxy and tert-butoxy.

The term "haloalkoxy" refers to a haloalkyl group, as defined herein, appended to the parent molecular moiety through an oxygen atom. In some embodiments, the alkoxy moiety has 1 to 8 carbon atoms ("C₁₋₈ haloalkoxy"). In some embodiments, the alkoxy moiety has 1 to 6 carbon atoms ("C₁₋₆ haloalkoxy"). In some embodiments, the alkoxy moiety has 1 to 4 carbon atoms ("C₁₋₄ haloalkoxy"). In some embodiments, the alkoxy moiety has 1 to 3 carbon atoms ("C₁₋₃ haloalkoxy"). In some embodiments, the alkoxy moiety has 1 to 2 carbon atoms ("C₁₋₂ haloalkoxy"). Representative examples of haloalkoxy include, but are not limited to, difluoromethoxy, trifluoromethoxy, and 2,2,2-trifluoroethoxy.

The term "alkoxyalkyl" is a substituted alkyl group, wherein one or more of the hydrogen atoms are independently replaced by an alkoxy group, as defined herein. In some embodiments, the alkoxyalkyl moiety has 1 to 8 carbon atoms ("C₁₋₈ alkoxyalkyl"). In some embodiments, the alkoxyalkyl moiety has 1 to 6 carbon atoms ("C₁₋₆ alkoxyalkyl"). In some embodiments, the alkoxyalkyl moiety has 1 to 4 carbon atoms ("C₁₋₄ alkoxyalkyl"). In some embodiments, the alkoxyalkyl moiety has 1 to 3 carbon atoms ("C₁₋₃ alkoxyalkyl"). In some embodiments, the alkoxyalkyl moiety has 1 to 2 carbon atoms ("C₁₋₂ alkoxyalkyl").

The term "heteroalkyl" refers to an alkyl group, which further includes at least one heteroatom (e.g., 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkyl group refers to a saturated group having from 1 to 20 carbon atoms and 1 or more heteroatoms within the parent chain ("heteroC₁₋₂₀ alkyl"). In some embodiments, a heteroalkyl group is a saturated group having 1 to 18 carbon atoms and 1 or more heteroatoms within the parent chain ("heteroC₁₋₁₈ alkyl"). In some embodiments, a heteroalkyl group is a saturated group having 1 to 16 carbon atoms and 1 or more heteroatoms within the parent chain ("heteroC₁₋₁₆ alkyl"). In some embodiments, a heteroalkyl group is a saturated group having 1 to 14 carbon atoms and 1 or more heteroatoms within the parent chain ("heteroC₁₋₁₄ alkyl"). In some embodiments, a heteroalkyl group is a saturated group having 1 to12 carbon atoms and 1 or more heteroatoms within the parent chain ("heteroC₁₋₁₂ alkyl"). In some embodiments, a heteroalkyl group is a saturated group having 1to 10 carbon atoms and 1 or more heteroatoms within the parent chain ("heteroC₁₋₁₀ alkyl"). In some embodiments, a heteroalkyl group is a saturated group having 1 to 8 carbon atoms and 1 or more heteroatoms within the parent chain ("heteroC₁₋₈ alkyl"). In some embodiments, a heteroalkyl group is a saturated group having 1 to 6 carbon atoms and 1 or more heteroatoms within the parent chain ("heteroC₁₋₆ alkyl"). In some embodiments, a heteroalkyl group is a saturated group having 1 to 4 carbon atoms and 1 or 2 heteroatoms within the parent chain ("heteroC₁₋₄ alkyl"). In some embodiments, a heteroalkyl group is a saturated group having 1 to 3 carbon atoms and 1 heteroatom within the parent chain ("heteroC₁₋₃ alkyl"). In some embodiments, a heteroalkyl group is a saturated group having 1 to 2 carbon atoms and 1 heteroatom within the parent chain ("heteroC₁₋₂ alkyl"). In some embodiments, a heteroalkyl group is a saturated group having 1 carbon atom and 1 heteroatom ("heteroC₁ alkyl"). In some embodiments, the heteroalkyl group defined herein is a partially unsaturated group having 1 or more heteroatoms within the parent chain and at least one unsaturated carbon, such as a carbonyl group. For example, a heteroalkyl group may comprise an amide or ester functionality in its parent chain such that one or more carbon atoms are unsaturated carbonyl groups. Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₂₀ alkyl. In certain embodiments, the heteroalkyl group is an unsubstituted heteroC₁₋₁₀ alkyl. In certain embodiments, the heteroalkyl group is a substituted heteroC₁₋₂₀ alkyl. In certain embodiments, the heteroalkyl group is an unsubstituted heteroC₁₋₁₀ alkyl.

The term "alkenyl" refers to a radical of a straight-chain or branched hydrocarbon group having from 2 to 10 carbon atoms and one or more carbon-carbon double bonds (e.g., 1, 2, 3, or 4 double bonds). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently unsubstituted (an "unsubstituted alkenyl") or substituted (a "substituted alkenyl") with one or more substituents. In certain embodiments, the alkenyl group is an unsubstituted C₂₋₁₀ alkenyl. In certain embodiments, the alkenyl group is a substituted C₂₋₁₀ alkenyl. In an alkenyl group, a C=C double bond for which the stereochemistry is not specified (e.g., -CH=CHCH₃ or 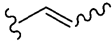 ) may be an (E)- or (Z)-double bond.

The term "heteroalkenyl" refers to an alkenyl group, which further includes at least one heteroatom (e.g., 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkenyl group refers to a group having from 2 to 10 carbon atoms, at least one double bond, and 1 or more heteroatoms within the parent chain ("heteroC₂₋₁₀ alkenyl"). In some embodiments, a heteroalkenyl group has 2 to 9 carbon atoms at least one double bond, and 1 or more heteroatoms within the parent chain ("heteroC₂₋₉ alkenyl").

In some embodiments, a heteroalkenyl group has 2 to 8 carbon atoms, at least one double bond, and 1 or more heteroatoms within the parent chain ("heteroC₂₋₈ alkenyl"). In some embodiments, a heteroalkenyl group has 2 to 7 carbon atoms, at least one double bond, and 1 or more heteroatoms within the parent chain ("heteroC₂₋₇ alkenyl"). In some embodiments, a heteroalkenyl group has 2 to 6 carbon atoms, at least one double bond, and 1 or more heteroatoms within the parent chain ("heteroC₂₋₆ alkenyl"). In some embodiments, a heteroalkenyl group has 2 to 5 carbon atoms, at least one double bond, and 1 or 2 heteroatoms within the parent chain ("heteroC₂₋₅ alkenyl"). In some embodiments, a heteroalkenyl group has 2 to 4 carbon atoms, at least one double bond, and 1 or 2 heteroatoms within the parent chain ("heteroC₂₋₄ alkenyl"). In some embodiments, a heteroalkenyl group has 2 to 3 carbon atoms, at least one double bond, and 1 heteroatom within the parent chain ("heteroC₂₋₃ alkenyl"). In some embodiments, a heteroalkenyl group has 2 to 6 carbon atoms, at least one double bond, and 1 or 2 heteroatoms within the parent chain ("heteroC₂₋₆ alkenyl"). Unless otherwise specified, each instance of a heteroalkenyl group is independently unsubstituted (an "unsubstituted heteroalkenyl") or substituted (a "substituted heteroalkenyl") with one or more substituents. In certain embodiments, the heteroalkenyl group is an unsubstituted heteroC₂₋₁₀ alkenyl. In certain embodiments, the heteroalkenyl group is a substituted heteroC₂₋₁₀ alkenyl.

The term "alkynyl" refers to a radical of a straight-chain or branched hydrocarbon group having from 2 to 10 carbon atoms and one or more carbon-carbon triple bonds (e.g., 1, 2, 3, or 4 triple bonds) ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkynyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently unsubstituted (an "unsubstituted alkynyl") or substituted (a "substituted alkynyl") with one or more substituents. In certain embodiments, the alkynyl group is an unsubstituted C₂₋₁₀ alkynyl. In certain embodiments, the alkynyl group is a substituted C₂₋₁₀ alkynyl.

The term "heteroalkynyl" refers to an alkynyl group, which further includes at least one heteroatom (e.g., 1, 2, 3, or 4 heteroatoms) selected from oxygen, nitrogen, or sulfur within (i.e., inserted between adjacent carbon atoms of) and/or placed at one or more terminal position(s) of the parent chain. In certain embodiments, a heteroalkynyl group refers to a group having from 2 to 10 carbon atoms, at least one triple bond, and 1 or more heteroatoms within the parent chain ("heteroC₂₋₁₀ alkynyl"). In some embodiments, a heteroalkynyl group has 2 to 9 carbon atoms, at least one triple bond, and 1 or more heteroatoms within the parent chain ("heteroC₂₋₉ alkynyl"). In some embodiments, a heteroalkynyl group has 2 to 8 carbon atoms, at least one triple bond, and 1 or more heteroatoms within the parent chain ("heteroC₂₋₈ alkynyl"). In some embodiments, a heteroalkynyl group has 2 to 7 carbon atoms, at least one triple bond, and 1 or more heteroatoms within the parent chain ("heteroC₂₋₇ alkynyl"). In some embodiments, a heteroalkynyl group has 2 to 6 carbon atoms, at least one triple bond, and 1 or more heteroatoms within the parent chain ("heteroC₂₋₆ alkynyl"). In some embodiments, a heteroalkynyl group has 2 to 5 carbon atoms, at least one triple bond, and 1 or 2 heteroatoms within the parent chain ("heteroC₂₋₅ alkynyl"). In some embodiments, a heteroalkynyl group has 2 to 4 carbon atoms, at least one triple bond, and 1 or 2 heteroatoms within the parent chain ("heteroC₂₋₄ alkynyl"). In some embodiments, a heteroalkynyl group has 2 to 3 carbon atoms, at least one triple bond, and 1 heteroatom within the parent chain ("heteroC₂₋₃ alkynyl"). In some embodiments, a heteroalkynyl group has 2 to 6 carbon atoms, at least one triple bond, and 1 or 2 heteroatoms within the parent chain ("heteroC₂₋₆ alkynyl"). Unless otherwise specified, each instance of a heteroalkynyl group is independently unsubstituted (an "unsubstituted heteroalkynyl") or substituted (a "substituted heteroalkynyl") with one or more substituents. In certain embodiments, the heteroalkynyl group is an unsubstituted heteroC₂₋₁₀ alkynyl. In certain embodiments, the heteroalkynyl group is a substituted heteroC₂₋₁₀ alkynyl.

The term "carbocyclyl" or "carbocyclic" refers to a radical of a non-aromatic cyclic hydrocarbon group having from 3 to 14 ring carbon atoms ("C₃₋₁₄ carbocyclyl") and zero heteroatoms in the non-aromatic ring system. In some embodiments, a carbocyclyl group has 3 to 10 ring carbon atoms ("C₃₋₁₀ carbocyclyl"). In some embodiments, a carbocyclyl group has 3 to 8 ring carbon atoms ("C₃₋₈ carbocyclyl"). In some embodiments, a carbocyclyl group has 3 to 7 ring carbon atoms ("C₃₋₇ carbocyclyl"). In some embodiments, a carbocyclyl group has 3 to 6 ring carbon atoms ("C₃₋₆ carbocyclyl"). In some embodiments, a carbocyclyl group has 4 to 6 ring carbon atoms ("C₄₋₆ carbocyclyl"). In some embodiments, a carbocyclyl group has 5 to 6 ring carbon atoms ("C₅₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₈ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl(C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or polycyclic (e.g., containing a fused, bridged or spiro ring system such as a bicyclic system ("bicyclic carbocyclyl") or tricyclic system ("tricyclic carbocyclyl")) and can be saturated or can contain one or more carbon-carbon double or triple bonds. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently unsubstituted (an "unsubstituted carbocyclyl") or substituted (a "substituted carbocyclyl") with one or more substituents. In certain embodiments, the carbocyclyl group is an unsubstituted C₃₋₁₄ carbocyclyl. In certain embodiments, the carbocyclyl group is a substituted C₃₋₁₄ carbocyclyl.

In some embodiments, "cycloalkyl" is a saturated monocyclic ("monocyclic cycloalkyl") or polycyclic (e.g., containing a fused, bridged or spiro ring system such as a bicyclic system ("bicyclic cycloalkyl") or tricyclic system ("tricyclic cycloalkyl ")) carbocyclyl group having from 3 to 14 ring carbon atoms ("C₃₋₁₄ cycloalkyl"). In some embodiments, a cycloalkyl group has 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 4 to 6 ring carbon atoms ("C₄₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₆). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Exemplary polycyclic cycloalkyls include bicyclo[2.2.1]heptanyl, bicyclo[2.2.2]octanyl, spiro[4.5]decanyl, cubanyl, and the like. Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is an unsubstituted C₃₋₁₄ cycloalkyl. In certain embodiments, the cycloalkyl group is a substituted C₃₋₁₄ cycloalkyl.

The term "heterocyclyl" or "heterocyclic" refers to a radical of a 3- to 14-membered non-aromatic ring system having ring carbon atoms and 1 to 4 ring heteroatoms, wherein each heteroatom is independently selected from nitrogen, oxygen, and sulfur ("3-14 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or polycyclic *(e.g.,* a fused, bridged or spiro ring system such as a bicyclic system ("bicyclic heterocyclyl") or tricyclic system ("tricyclic heterocyclyl")), and can be saturated or can contain one or more carbon-carbon or carbon-heteroatom double or triple bonds. Heterocyclyl polycyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently unsubstituted (an "unsubstituted heterocyclyl") or substituted (a "substituted heterocyclyl") with one or more substituents. In certain embodiments, the heterocyclyl group is an unsubstituted 3-14 membered heterocyclyl. In certain embodiments, the heterocyclyl group is a substituted 3-14 membered heterocyclyl.

In some embodiments, a heterocyclyl group is a 4-10 membered non-aromatic ring system having ring carbon atoms and 1-4 ring heteroatoms, wherein each heteroatom is independently selected from nitrogen, oxygen, and sulfur ("4-10 membered heterocyclyl"). In some embodiments, a heterocyclyl group is a 4-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has 1 ring heteroatom selected from nitrogen, oxygen, and sulfur.

Exemplary 3-membered heterocyclyl groups containing 1 heteroatom include, without limitation, aziridinyl, oxiranyl, and thiiranyl. Exemplary 4-membered heterocyclyl groups containing 1 heteroatom include, without limitation, azetidinyl, oxetanyl, and thietanyl. Exemplary 5-membered heterocyclyl groups containing 1 heteroatom include, without limitation, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, without limitation, dioxolanyl, oxathiolanyl, and dithiolanyl. Exemplary 5-membered heterocyclyl groups containing 3 heteroatoms include, without limitation, triazolinyl, oxadiazolinyl, and thiadiazolinyl. Exemplary 6-membered heterocyclyl groups containing 1 heteroatom include, without limitation, piperidinyl, tetrahydropyranyl, dihydropyridinyl, and thianyl. Exemplary 6-membered heterocyclyl groups containing 2 heteroatoms include, without limitation, piperazinyl, morpholinyl, dithianyl, and dioxanyl. Exemplary 6-membered heterocyclyl groups containing 3 heteroatoms include, without limitation, triazinyl. Exemplary 7-membered heterocyclyl groups containing 1 heteroatom include, without limitation, azepanyl, oxepanyl, and thiepanyl. Exemplary 8-membered heterocyclyl groups containing 1 heteroatom include, without limitation, azocanyl, oxecanyl, and thiocanyl. Exemplary bicyclic heterocyclyl groups include, without limitation, indolinyl, isoindolinyl, dihydrobenzofuranyl, dihydrobenzothienyl, tetrahydrobenzothienyl, tetrahydrobenzofuranyl, tetrahydroindolyl, tetrahydroquinolinyl, tetrahydroisoquinolinyl, decahydroquinolinyl, decahydroisoquinolinyl, octahydrochromenyl, octahydroisochromenyl, decahydronaphthyridinyl, decahydro-1,8-naphthyridinyl, octahydropyrrolo[3,2-b]pyrrole, indolinyl, phthalimidyl, naphthalimidyl, chromanyl, chromenyl, l*H*-benzo[*e*][1,4]diazepinyl, 1,4,5,7-tetrahydropyrano[3,4-b]pyrrolyl, 5,6-dihydro-4H-furo[3,2-b]pyrrolyl, 6,7-dihydro-5H-furo[3,2-b]pyranyl, 5,7-dihydro-4H-thieno[2,3-c]pyranyl, 2,3-dihydro-1H-pyrrolo[2,3-b]pyridinyl, 2,3-dihydrofuro[2,3-b]pyridinyl, 4,5,6,7-tetrahydro-1H-pyrrolo[2,3-b]pyridinyl, 4,5,6,7-tetrahydrofuro[3,2-c]pyridinyl, 4,5,6,7-tetrahydrothieno[3,2-b]pyridinyl, 1,2,3,4-tetrahydro-1,6-naphthyridinyl, and the like.

The term "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has 6 ring carbon atoms ("C₆ aryl"; *e.g.,* phenyl). In some embodiments, an aryl group has 10 ring carbon atoms ("C₁₀ aryl"; *e.g.,* naphthyl such as 1-naphthyl and 2-naphthyl). In some embodiments, an aryl group has 14 ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently unsubstituted (an "unsubstituted aryl") or substituted (a "substituted aryl") with one or more substituents. In certain embodiments, the aryl group is an unsubstituted C₆₋₁₄ aryl. In certain embodiments, the aryl group is a substituted C₆₋₁₄ aryl.

"Aralkyl" is a subset of "alkyl" and refers to an alkyl group substituted by an aryl group, wherein the point of attachment is on the alkyl moiety.

The term "heteroaryl" refers to a radical of a 5-14 membered monocyclic or polycyclic *(e.g.,* bicyclic, tricyclic) 4n+2 aromatic ring system *(e.g.,* having 6, 10, or 14 π electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl polycyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused polycyclic (aryl/heteroaryl) ring system. Polycyclic heteroaryl groups wherein one ring does not contain a heteroatom (e.g., indolyl, quinolinyl, carbazolyl, and the like) the point of attachment can be on either ring, *i.e.,* either the ring bearing a heteroatom (e.g., 2-indolyl) or the ring that does not contain a heteroatom (e.g., 5-indolyl).

In some embodiments, a heteroaryl group is a 5-12 membered aromatic ring system having ring carbon atoms and 1-4 ring heteroatoms provided in the aromatic ring system, wherein each heteroatom is independently selected from nitrogen, oxygen, and sulfur ("5-12 membered heteroaryl"). In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently unsubstituted (an "unsubstituted heteroaryl") or substituted (a "substituted heteroaryl") with one or more substituents. In certain embodiments, the heteroaryl group is an unsubstituted 5-14 membered heteroaryl. In certain embodiments, the heteroaryl group is a substituted 5-14 membered heteroaryl.

Exemplary 5-membered heteroaryl groups containing 1 heteroatom include, without limitation, pyrrolyl, furanyl, and thiophenyl. Exemplary 5-membered heteroaryl groups containing 2 heteroatoms include, without limitation, imidazolyl, pyrazolyl, oxazolyl, isoxazolyl, thiazolyl, and isothiazolyl. Exemplary 5-membered heteroaryl groups containing 3 heteroatoms include, without limitation, triazolyl, oxadiazolyl, and thiadiazolyl. Exemplary 5-membered heteroaryl groups containing 4 heteroatoms include, without limitation, tetrazolyl. Exemplary 6-membered heteroaryl groups containing 1 heteroatom include, without limitation, pyridinyl. Exemplary 6-membered heteroaryl groups containing 2 heteroatoms include, without limitation, pyridazinyl, pyrimidinyl, and pyrazinyl. Exemplary 6-membered heteroaryl groups containing 3 or 4 heteroatoms include, without limitation, triazinyl and tetrazinyl, respectively. Exemplary 7-membered heteroaryl groups containing 1 heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl. Exemplary tricyclic heteroaryl groups include, without limitation, phenanthridinyl, dibenzofuranyl, carbazolyl, acridinyl, phenothiazinyl, phenoxazinyl, and phenazinyl.

"Heteroaralkyl" is a subset of "alkyl" and refers to an alkyl group substituted by a heteroaryl group, wherein the point of attachment is on the alkyl moiety.

The term "unsaturated bond" refers to a double or triple bond.

The term "unsaturated" or "partially unsaturated" refers to a moiety that includes at least one double or triple bond.

The term "saturated" refers to a moiety that does not contain a double or triple bond, *i.e.,* the moiety only contains single bonds.

Affixing the suffix "-ene" to a group indicates the group is a divalent moiety, e.g., alkylene is the divalent moiety of alkyl, alkenylene is the divalent moiety of alkenyl, alkynylene is the divalent moiety of alkynyl, heteroalkylene is the divalent moiety of heteroalkyl, heteroalkenylene is the divalent moiety of heteroalkenyl, heteroalkynylene is the divalent moiety of heteroalkynyl, carbocyclylene is the divalent moiety of carbocyclyl, heterocyclylene is the divalent moiety of heterocyclyl, arylene is the divalent moiety of aryl, and heteroarylene is the divalent moiety of heteroaryl.

A group is optionally substituted unless expressly provided otherwise. The term "optionally substituted" refers to being substituted or unsubstituted. In certain embodiments, alkyl, alkenyl, alkynyl, alkoxy, heteroalkyl, heteroalkenyl, heteroalkynyl, carbocyclyl, cycloalkyl, heterocyclyl, aryl, and heteroaryl groups are optionally substituted. "Optionally substituted" refers to a group which may be substituted or unsubstituted (e.g., "substituted" or "unsubstituted" alkyl, "substituted" or "unsubstituted" alkenyl, "substituted" or "unsubstituted" alkynyl, "substituted" or "unsubstituted" heteroalkyl, "substituted" or "unsubstituted" heteroalkenyl, "substituted" or "unsubstituted" heteroalkynyl, "substituted" or "unsubstituted" carbocyclyl, "substituted" or "unsubstituted" heterocyclyl, "substituted" or "unsubstituted" aryl, or "substituted" or "unsubstituted" heteroaryl group). In general, the term "substituted" means that at least one hydrogen present on a group is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, and includes any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety. The invention is not intended to be limited in any manner by the exemplary substituents described herein. Exemplary carbon atom substituents include, but are not limited to, halogen, -CN, -NO₂, -N₃, - SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, - C(=O)R^{aa}, -CO₂H, -CHO, -C(OR^{cc})₃, -CO₂R^{aa}, -OC(=O)R^{aa}, -OCO₂R^{aa}, -C(=O)N(R^{bb})₂, - OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, - C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, - NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, - OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, - C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)(R^{aa})₂, -P(=O)(OR^{cc})₂, -OP(=O)(R^{aa})₂, -OP(=O)(OR^{cc})₂, -P(=O)(N(R^{bb})₂)₂,-OP(=O)(N(R^{bb})₂)₂, - NR^{bb}P(=O)(R^{aa})₂, -NR^{bb}P(=O)(OR^{cc})₂, -NR^{bb}P(=O)(N(R^{bb})₂)₂, -P(R^{cc})₂, -P(OR^{cc})₂, -P(R^{cc})₃⁺X⁻, - P(OR^{cc})₃⁺X⁻, -P(R^{cc})₄, -P(OR^{cc})₂, -OP(R^{cc})₂, -OP(R^{cc})₃⁺X⁻, -OP(OR^{cc})₂, -OP(OR^{cc})₃⁺X⁻, - OP(R^{cc})₄, -OP(OR^{cc})₄, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C_{2- 10} alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc}; each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; each instance of R^{bb} is, independently, selected from hydrogen, -OH, -OR^{aa}, -N(R^{cc})₂, -CN, -C(=O)R^{aa}, -C(=O)N(R^{cc})₂, - CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, - C(=S)N(R^{cc})₂, -C(=O)SR^{cc}, -C(=S)SR^{cc}, -P(=O)(R^{aa})₂, -P(=O)(OR^{cc})₂, -P(=O)(N(R^{cc})₂)₂, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion; each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; each instance of R^{dd} is, independently, selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, - N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, - C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, - C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, - Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)(OR^{ee})₂, - P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S; wherein X⁻ is a counterion; each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-10 membered heterocyclyl or 5-10 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; and each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X-, -NH(C₁₋₆ alkyl)2⁺ X⁻, -NH₂(C₁₋₆ alkyl)⁺X⁻, -NH₃⁺X⁻, -N(OC₁₋₆ alkyl)(C₁₋₆ alkyl), - N(OH)(C₁₋₆ alkyl), -NH(OH), -SH, -SC₁₋₆ alkyl, -SS(C₁₋₆ alkyl), -C(=O)(C₁₋₆ alkyl), -CO₂H, - CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)(C₁₋₆ alkyl), -N(C₁₋₆ alkyl)C(=O)( C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(=NH)NH(C₁₋₆ alkyl), -OC(=NH)NH₂, - NHC(=NH)N(C₁₋₆ alkyl)₂, -NHC(=NH)NH₂, -NHSO₂(C₁₋₆ alkyl), -SO₂N(C₁₋₆ alkyl)₂, - SO₂NH(C₁₋₆ alkyl), -SO₂NH₂, -SO₂(C₁₋₆ alkyl), -SO₂O(C₁₋₆ alkyl), -OSO₂(C₁₋₆ alkyl), -SO(C₁₋₆ alkyl), -Si(C₁₋₆ alkyl)₃, -OSi(C₁₋₆ alkyl)₃, -C(=S)N(C₁₋₆ alkyl)₂, -C(=S)NH(C₁₋₆ alkyl), -C(=S)NH₂, -C(=O)S(C₁₋₆ alkyl), -C(=S)SC₁₋₆ alkyl, -SC(=S)SC₁₋₆ alkyl, -P(=O)(OC₁₋₆ alkyl)₂, -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆ alkyl, heteroC₂₋₆ alkenyl, heteroC₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

The term "halo" or "halogen" refers to fluorine (fluoro, -F), chlorine (chloro, -Cl), bromine (bromo, -Br), or iodine (iodo, -I).

The term "hydroxyl" or "hydroxy" refers to the group -OH. The term "substituted hydroxyl" or "substituted hydroxyl," by extension, refers to a hydroxyl group wherein the oxygen atom directly attached to the parent molecule is substituted with a group other than hydrogen, and includes groups selected from -OR^{aa}, -ON(R^{bb})₂, -OC(=O)SR^{aa}, -OC(=O)R^{aa}, - OCO₂R^{aa}, -OC(=O)N(R^{bb})₂, -OC(=NR^{bb})R^{aa}, -OC(=NR^{bb})OR^{aa}, -OC(=NR^{bb})N(R^{bb})₂, - OS(=O)R^{aa}, -OSO₂R^{aa}, -OSi(R^{aa})₃, -OP(R^{cc})₂, -OP(R^{cc})₃⁺X⁻, -OP(OR^{cc})₂, -OP(OR^{cc})₃⁺X⁻, - OP(=O)(R^{aa})₂, -OP(=O)(OR^{cc})₂, and -OP(=O)(N(R^{bb})₂)₂, wherein X⁻, R^{aa}, R^{bb} and R^{cc} are as defined herein.

The term "oxo" refers to the group =O, and the term "thiooxo" refers to the group =S.

As used herein, the terms "administering" and "administration" refer to any method of providing a pharmaceutical preparation to a subject. Such methods are well known to those skilled in the art and include, but are not limited to, oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In some examples, a preparation can be administered therapeutically; that is, administered to treat an existing disease or condition. In some examples, a preparation can be administered prophylactically; that is, administered for prevention of a disease or condition.

The term "carrier" means a compound, composition, substance, or structure that, when in combination with a compound or composition, aids or facilitates preparation, storage, administration, delivery, effectiveness, selectivity, or any other feature of the compound or composition for its intended use or purpose. For example, a carrier can be selected to minimize any degradation of the active ingredient and to minimize any adverse side effects in the subject.

As used herein, the term "diagnosed" means having been subjected to a physical examination by a person of skill, for example, a physician, and found to have a condition that can be diagnosed or treated by the compounds, compositions, or methods disclosed herein. For example, "diagnosed with arterial thrombosis" means having been subjected to a physical examination by a person of skill, for example, a physician, and found to have a condition that can be diagnosed or treated by a compound or composition that can treat or prevent arterial thrombosis.

As used herein, the terms "effective amount" and "amount effective" refer to an amount that is sufficient to achieve the desired result or to have an effect on an undesired condition. For example, a "therapeutically effective amount" refers to an amount that is sufficient to achieve the desired therapeutic result or to have an effect on undesired symptoms but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. Dosage can vary and can be administered in one or more dose administrations daily, for one or several days. Guidance can be found in the literature for appropriate dosages for given classes of pharmaceutical products. In some examples, a preparation can be administered in a "prophylactically effective amount"; that is, an amount effective for prevention of a disease or condition.

As used herein, the phrase "identified to be in need of treatment for a disorder," or the like, refers to selection of a subject based upon need for treatment of the disorder. For example, a subject can be identified as having a need for treatment of a disorder (e.g., a disorder related to thrombosis) based upon an earlier diagnosis by a person of skill and thereafter subjected to treatment for the disorder. It is contemplated that the identification can, in some examples, be performed by a person different from the person making the diagnosis. It is also contemplated, in some examples, that the administration can be performed by one who subsequently performed the administration. "Optional" or "optionally" means that the subsequently described event or circumstance can or cannot occur and that the description includes instances where the event or circumstance occurs and instances where it does not.

The term "pharmaceutically acceptable" describes a material that is not biologically or otherwise undesirable, i.e., without causing an unacceptable level of undesirable biological effects or interacting in a deleterious manner.

As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactidepolyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose.

The term "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.

By "reduce" or other forms of the word, such as "reducing" or "reduction," it is meant lowering of an event or characteristic (e.g., thrombi formation). It is understood that this is typically in relation to some standard or expected value. In other words, it is relative, but it is not always necessary for the standard or relative value to be referred to. For example, "reduces thrombi formation" means decreasing the number of thrombi cells relative to a standard or a control.

The term "substantially" is to be construed as a term of approximation. The term "substantially free", "free", "free from", "substantially free of", or "free of" refers to compositions completely lacking the component or having such a small amount of the component that the component does not affect the performance of the composition. In some cases, the component may be present as an impurity or as a contaminant or as a very small amount, e.g., less than 10%, or less than 5 %, or less than 0.5 %. In another embodiment, the amount of the component is less than 0.1 % and in yet another embodiment, the amount of component is less than 0.01 %, less than 0.001 %, less than 0.0001 %, or less than 0.00001 %.

The term "von Willebrand factor" as used herein includes naturally occurring (native) VWF, but also variants thereof retaining at least some of the FVIII binding activity of naturally occurring VWF, e.g. sequence variants where one or more residues have been inserted, deleted or substituted.

The term "platelet" can include whole platelets, fragmented platelets, platelet derivatives, or thrombosomes. In some embodiments, the thrombus comprises a substantial amount of platelet cells.

The term "fibrin," as used herein refers to a fibrous protein involved in the clotting of blood. In some embodiments it is a fibrillar protein that is polymerized to form a "mesh" that forms a hemostatic plug or clot (e.g., in conjunction with platelets). Fibrin is involved in signal transduction, blood coagulation, platelet activation, and protein polymerization.

The term "anticoagulant" is meant to refer to any agent capable of prolonging the prothrombin and partial thromboplastin time tests and reducing the levels of prothrombin and factors VII, IX and X. In some embodiments, anticoagulants include coumarin derivatives and heparin as well as aspirin, which may also be referred to as an antiplatelet agent.

In some embodiments, the compounds of the disclosure are administered with a second therapeutic agent, such as a thrombolytic (or lytic) agent. As used herein, the terms "thrombolytic agent" or "lytic agent" refer to any agent capable of inducing reperfusion by dissolving, dislodging or otherwise breaking up a clot, e.g., by either dissolving a clot, or inhibiting the formation of such a clot. Reperfusion occurs when the clot is dissolved and blood flow is restored. Exemplary thrombolytic agents include, but are not limited to, tissue-type plasminogen activator (t-PA), streptokinase (SK), prourokinase, urokinase (uPA), alteplase (also known as Activase^{®}, Genentech, Inc.), reteplase (also known as r-PA or retavase^{®}, Centocor, Inc.), tenecteplase (also known as TNKTM, Genentech, Inc.), Streptase^{®} (AstraZeneca, LP), lanoteplase (Bristol-Myers Squibb Company), monteplase (Eisai Company, Ltd.), saruplase (also known as r-scu-PA and rescupaseTM, Gmnenthal GmbH, Corp.), staphylokinase, and anisoylated plasminogen-streptokinase activator complex (also known as APSAC, Anistreplase and Eminase^{®}, SmithKline Beecham Corp.). Thrombolytic agents also include other genetically engineered plasminogen activators. The invention can additionally employ hybrids, physiologically active fragments or mutant forms of the above thrombolytic agents. The term "tissue-type plasminogen activator" as used herein is intended to include such hybrids, fragments and mutants, as well as both naturally derived and recombinantly derived tissue-type plasminogen activator.

As used herein, the term "subject" refers to the target of administration, e.g., patient. Thus, the subject of the herein disclosed methods can be a vertebrate, such as a mammal, a fish, a bird, a reptile, or an amphibian. Alternatively, the subject of the herein disclosed methods can be a human, non-human primate, horse, pig, rabbit, dog, sheep, goat, cow, cat, guinea pig, rodent, or fruit fly. The term does not denote a particular age or sex. Thus, adult and newborn subjects, as well as fetuses, whether male or female, are intended to be covered. In some examples, the subject is a mammal. A patient refers to a subject afflicted with a disease or disorder. The term "patient" includes human and veterinary subjects. In some examples of the disclosed methods, the subject has been diagnosed with a need for treatment or prevention of thrombosis.

As used herein, the term "treatment" or "treating" refers to the medical management of a patient with the intent to cure, ameliorate, reduce, or stabilize a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; and supportive treatment, that is, treatment employed to supplement another specific therapy directed toward the improvement of the associated disease, pathological condition, or disorder.

### Arterial Thrombosis

Disclosed are methods and compositions for treating and preventing arterial thrombosis, and reducing thrombi in arteries. Arterial thrombosis is the process which forms large, occlusive blood clot in arteries. These clots impede blood flow to downstream tissue, causing ischemic events such as heart attacks and strokes. Structurally, arterial thrombi are distinct from other clots such as venous thrombi and pulmonary emboli, which are comprised mainly of red blood cells (RBCs) and likely form through the coagulation pathway. The distinctive structure and composition of arterial and venous thrombi and pulmonary emboli. In contrast, arterial thrombi are made up of platelet-rich regions which can range from 11-99% of the total clot volume. The formation of platelet-rich regions is the result of a significant process of concentrating platelets, given that platelets are ~10x smaller and are at a ~20x lower concentration compared to RBCs. This formation of platelet-rich regions can be explained by platelet margination to the wall of vessels and selective capture of platelets onto the growing mural thrombus.

Arterial thrombi are platelet rich because they form through Shear Induced Platelet Aggregation (SIPA). In regions of pathologically high shear stress, the plasma protein von Willebrand Factor (VWF) activates and can bind to both collagen and platelets near the vessel wall. Captured platelets form a new boundary that defines flow and release additional VWF, increasing the local VWF concentration by ~50x and capturing additional nearby platelets. This kicks off a positive feedback loop which ends with the formation of a large, occlusive clot.

Targeting VWF instead of platelets is an alternative mechanism for reducing arterial thrombosis. Without being bound by theory, presently disclosed are methods of treating or preventing arterial thrombosis in a subject in need thereof comprise administering to the subject a compound disclosed herein (e.g., compound of structural formula (I) or (II)), which results in reduction of the S-S bond in the VWF fibers and dissolution of the thrombus. Methods of preventing arterial thrombi formation, e.g., shear induced platelet aggregation, comprise administering to the artery a compound disclosed herein (e.g., compound of structural formula (I) or (II)).

In the disclosed methods, the arterial thrombus being treated, prevented or reduced is a white thrombus that is characterized by a predominance of platelets and/or von Willebrand Factor (VWF), and, in some cases, a paucity of red blood cells. In certain examples, the thrombus is substantially free of red blood cells. In some examples, the thrombus has a concentration of red blood cells of less than about 30%, or less than 25%, or less than 20% or less than 15% or less than 10%, or less than about 5%, or less than about 1%, or less than 0.5%.

### Administration

The compound disclosed herein (e.g., compounds of structural formula (I) or (II)) can be administered sequentially or simultaneously in separate or combined pharmaceutical formulations. When one or more of the compounds are combined with a second therapeutic agent, the dose of each compound can be either the same or differ from that when the compound is used alone. Appropriate doses will be readily appreciated by those skilled in the art.

*In vivo* application of the compounds disclosed herein (e.g., compound of structural formula (I) or (II)) and compositions containing them can be accomplished by any suitable method and technique presently or prospectively known to those skilled in the art. For example, the compounds can be formulated in a physiologically- or pharmaceutically-acceptable form and administered by any suitable route known in the art, including oral, nasal, rectal, topical, and parenteral routes of administration. As used herein, the term parenteral includes subcutaneous, intradermal, intravenous, intramuscular, intraperitoneal, and intrastemal administration, such as by injection. Administration of the compounds disclosed herein (e.g., compounds of structural formula (I) or (II)) or compositions can be a single administration or at continuous or distinct intervals as readily determined by a person skilled in the art.

The compounds disclosed herein and compositions comprising them can also be administered utilizing liposome technology, slow-release capsules, implantable pumps, and biodegradable containers. These delivery methods can provide a uniform dosage over an extended period. The compounds can also be administered in their salt derivative forms or crystalline forms. The compounds disclosed herein can be formulated according to known methods for preparing pharmaceutically acceptable compositions. Formulations are described in detail in many sources which are well known and readily available to those skilled in the art. For example, Remington's Pharmaceutical Science by E.W. Martin (1995) describes formulations that can be used in connection with the disclosed methods. In general, the compounds disclosed herein can be formulated such that an effective amount of the compound is combined with a suitable carrier to facilitate the effective administration of the compound. The compositions used can also be in a variety of forms. These include, for example, solid, semisolid, and liquid dosage forms, such as tablets, pills, powders, liquid solutions or suspension, suppositories, injectable and infusible solutions, and sprays. The preferred form depends on the intended mode of administration and therapeutic application. The compositions also preferably include conventional pharmaceutically-acceptable carriers and diluents known to those skilled in the art. Examples of carriers or diluents for use with the compounds include ethanol, dimethyl sulfoxide, glycerol, alumina, starch, saline, and equivalent carriers and diluents. To provide for the administration of such dosages for the desired treatment, compositions disclosed herein can advantageously comprise between about 0.1% and 99%, and especially, 1 and 15% by weight of the total of one or more of the subject compounds based on the weight of the total composition including carrier or diluent.

Formulations suitable for administration include, for example, aqueous sterile injection solutions, which can contain antioxidants, buffers, bacteriostats, and solutes that render the formulation isotonic with the blood of the intended recipient; and aqueous and nonaqueous sterile suspensions, which can include suspending agents and thickening agents. The formulations can be presented in unit-dose or multi-dose containers, for example, sealed ampoules and vials. The formulations can be stored in a freeze-dried (lyophilized) condition requiring only the condition of the sterile liquid carrier, for example, water for injections, before use. Extemporaneous injection solutions and suspensions can be prepared from sterile powder, granules, tablets, etc. It should be understood that in addition to the ingredients particularly mentioned above, the compositions disclosed herein can include other agents conventional in the art regarding the type of formulation in question.

Compounds disclosed herein and compositions comprising them can be delivered to a cell either through direct contact with the cell or via a carrier. Carriers for delivering compounds and compositions to cells are known in the art and include, for example, encapsulating the composition in a liposome moiety. The pharmaceutical dosage forms suitable for injection or infusion can include sterile aqueous solutions or dispersions or sterile powders comprising the active ingredient, which are adapted for the extemporaneous preparation of sterile injectable or infusible solutions or dispersions, optionally encapsulated in liposomes. The ultimate dosage form should be sterile, fluid, and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, non-toxic glyceryl esters, and suitable mixtures thereof. The proper fluidity can be maintained, for example, by the formation of liposomes, by the maintenance of the required particle size in the case of dispersions, or by the use of surfactants. Optionally, the prevention of the action of microorganisms can be brought about by various other antibacterial and antifungal agents, for example, parabens, chlorobutanol, phenol, sorbic acid, thimerosal, and the like. In many cases, it will be preferable to include isotonic agents, for example, sugars, buffers, or sodium chloride. Prolonged absorption of the injectable compositions can be brought about by including agents that delay absorption, such as aluminum monostearate and gelatin.

Sterile injectable solutions are prepared by incorporating a compound and/or agent disclosed herein in the required amount in the appropriate solvent with various other ingredients enumerated above, as required, followed by filter sterilization. In the case of sterile powders for the preparation of sterile injectable solutions, the preferred methods of preparation are vacuum drying and freeze-drying techniques, which yield a powder of the active ingredient plus any additional desired ingredient present in the previously sterile- filtered solutions.

In some embodiments of the disclosed treatment methods, the subject can be administered a dose of the compounds disclosed herein (e.g., compounds of structural formula (I) or (II)) as low as 1.25 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 57.5 mg, 60 mg, 62.5 mg, 65 mg, 67.5 mg, 70 mg, 72.5 mg, 75 mg, 77.5 mg, 80 mg, 82.5 mg, 85 mg, 87.5 mg, 90 mg, 100 mg, 200 mg, 500 mg, 1000 mg, or 2000 mg once daily, twice daily, three times daily, four times daily, once weekly, twice weekly, or three times per week in order to treat the disease or disorder in the subject. In some embodiments, the subject may be administered a dose of a compound as high as 1.25 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 57.5 mg, 60 mg, 62.5 mg, 65 mg, 67.5 mg, 70 mg, 72.5 mg, 75 mg, 77.5 mg, 80 mg, 82.5 mg, 85 mg, 87.5 mg, 90 mg, 100 mg, 200 mg, 500 mg, 1000 mg, or 2000 mg, once daily, twice daily, three times daily, four times daily, once weekly, twice weekly, or three times per week in order to treat the disease or disorder in the subject. Minimal and/or maximal doses of the compounds may include doses falling within dose ranges having as end-points any of these disclosed doses (e.g., 2.5 mg- 200 mg). In some examples, the subject is administered a single dose of the compound disclosed herein (e.g., compounds of structural formula (I) or (II)).

In some embodiments of the disclosed treatment methods, the subject can be administered a dose of the compound disclosed herein (e.g., compounds of structural formula (I) or (II)) sufficient to result in a concentration of the compound in the subject's blood of up to 5 mM, e.g., up to 1 mM, 1.5 mM, 2 mM, 2.5 mM, 3 mM, 3.5 mM, 4 mM, 4.5 mM or 5 mM, where any of the stated values can form an upper or lower endpoint of a range. In other examples, the subject can be administered a dose of the compound disclosed herein (e.g., compounds of structural formula (I) or (II)) sufficient to result in a concentration of the compound in the subject's blood of up to 10 mM, e.g., up to 5.5 mM, 6 mM, 6.5 mM, 7 mM, 7.5 mM, 8 mM, 8.5 mM, 9 mM, 9.5 mM, or 10 mM, wherein any of the stated values can form an upper or lower endpoint of a range.

In various embodiments, the compounds of this disclosure (e.g., a compound represented by any one of structural formulas (I)-(II) or a pharmaceutically acceptable salt thereof) can be used alone or in combination with other therapeutic agents. Combination therapies according to the present disclosure comprise the administration of at least one compound of the disclosure, and the use of at least one other therapeutically active agent. For example, combination therapies according to the present disclosure comprise the administration of at least one compound of the disclosure and at least one other therapeutically active agent to a subject in need of treatment for a given disease or disorder.

The compounds of the disclosure (e.g., a compound represented by any one of structural formulas (I)-(II) or a pharmaceutically acceptable salt thereof) and the other therapeutically active agent can be administered together in a single pharmaceutical composition or separately and, when administered separately this can occur simultaneously or sequentially in any order. The amounts of the compounds of the disclosure and other therapeutically active agents and the relative timings of administration can be selected in order to achieve the desired combined therapeutic effect. Thus in a further aspect, there is provided a combination comprising a compound of the disclosure together with one or more other therapeutically active agents.

In some embodiments, the one or more other therapeutically active agent is selected from the group consisting of anticoagulants, pro-coagulant antagonists, antiplatelet agents, thrombolytic agents, anti-thrombolytic agent antagonists, fibrinolytic enzymes, and any combinations thereof.

In some embodiments, the one or more other therapeutically active agent selected from the group consisting of tissue-type plasminogen activator (t-PA), streptokinase (SK), prourokinase, urokinase (uPA), recombinant tissue plasminogent activators (r-tPAs), alteplase, reteplase, lanoteplase,, saruplase (also known as r-scu-PA), staphylokinase, and anisoylated plasminogen-streptokinase activator complex (also known as APSAC), and any combinations thereof.

In some embodiments, the one or more other therapeutically active agent is selected from the group consisting of aspirin, wafarin (coumadin), acenocoumarol, ancrod, anisindione, bromindione, clorindione, coumetarol, cyclocumarol, dextran, dextran sulfate sodium, dicumarol, diphenadione, ethyl biscoumacetate, ethylidene dicoumarol, fluindione, heparin, hirudin, lyapolate sodium, oxazidione, pentosan polysulfate, phenindione, phenprocoumon, phosvitin, picotamide, tioclomarol, dipyridamole (persantin), sulfinpyranone (anturane), ticlopidine (ticlid), tissue plaminogen activator (activase), plasmin, pro-urokinase, urokinase (abbokinase) streptokinase (streptase), and anistreplase/APSAC (eminase).

In a 1st embodiment, the present disclosure relates to a method of treating a disease or disorder associated with thrombus formation in a subject in need thereof, comprising administering to the subject a therapeutically effective amount of a compound represented by structural formula (I) or structural formula (II) or a pharmaceutically acceptable salt thereof:

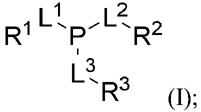

HO-M-SH (II),

wherein
each L¹, L², and L³ is independently C₁₋₆ alkylene;
M is C₂₋₆ alkylene;
each R¹, R², and R³ is independently selected from C(O)OR⁵, OR⁴, C(O)NR⁶R⁷, NR⁸C(O)R⁹, and NR¹⁰R¹¹,
each R⁴, R⁵, R⁶, R⁷, R⁸, R⁹, R¹⁰, and R¹¹ is selected from H and C₁₋₆ alkyl, or
R⁶ and R⁷, or R¹⁰ and R¹¹, together with the nitrogen atom to which they are attached form a 3- to 8-membered heterocyclyl,
wherein each C₁₋₆ alkylene, C₂₋₆ alkylene, and C₁₋₆ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
wherein
each R¹¹, R¹², R¹³, R¹⁴, R^{14*}, R¹⁵, R^{15*}, R¹⁶, R¹⁷, R^{16*}, R^{17*}, R¹⁸, R^{18a}, R¹⁹, R²⁰, R^{20a}, R^{20b}, R²¹, R²², R^{21*}, R^{22*}, R²³, R²⁴, R²⁵, R²⁶, and R²⁷ is independently selected from H, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl, or
one or more of the pairs of variables selected from R¹² and R¹³, R¹⁶ and R¹⁷, R^{16*} and R^{17*}, R²¹ and R²², R^{21*} and R^{22*}, and R²⁵ and R²⁶, together with the nitrogen to which they are attached, form a 3-8 membered ring, and
each of the substituents selected from C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl is further optionally substituted with 1 to 3 substituents independently selected from deuterium, oxo, F, Cl, Br, CN, NO₂, OR^{14*}, SR^{15*} , NR^{16*}R^{17*}, C₁₋₆ alkyl, C₁₋₆ haloalkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.

Embodiment 2. The method of embodiment 1, wherein the compound is represented by structural formula (I).
Embodiment 3. The method of embodiment 2, wherein L¹, L², and L³ are each C₂₋₃ alkylene, wherein the C₂₋₃ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 4. The method of any one of embodiments 1-3, wherein each R¹, R², and R³ is independently selected from C(O)NR⁶R⁷, NR⁸C(O)R⁹, and NR¹⁰R¹¹.
Embodiment 5. The method of any one of embodiments 1-3, wherein the compound is represented by structural formula (Ia): 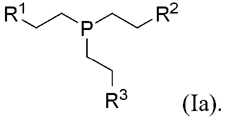
Embodiment 6. The method of embodiment 5, wherein the compound is represented by structural formula (Ib): 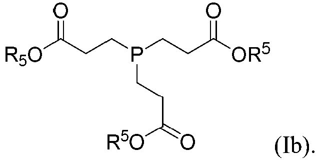
Embodiment 7. The method of embodiment 6, wherein each R⁵ is independently selected from H and C₁₋₃ alkyl, wherein each C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 8. The method of embodiment 7, wherein the compound is represented by one of the following structural formulas: 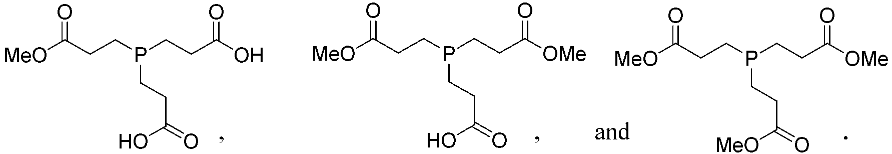
Embodiment 9. The method of embodiment 7, wherein the compound is represented by the following structural formula: 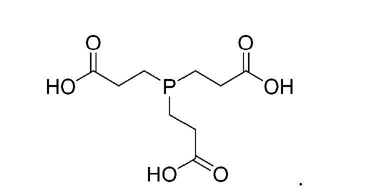
Embodiment 10. The method of any one of embodiments 1-3, wherein the compound is represented by structural formula (Ic): 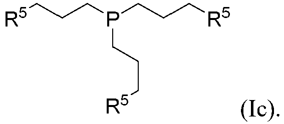
Embodiment 11. The method of embodiment 10, wherein the compound is represented by structural formula (Id): 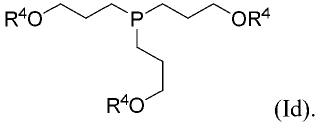
Embodiment 12. The method of embodiment 11, wherein each R⁴ is independently selected from H and C₁₋₃ alkyl, wherein the C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 13. The method of embodiment 12, wherein the compound is represented by the following structural formula: 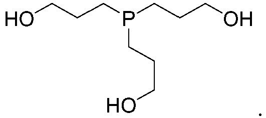
Embodiment 14. The method of embodiment 1, wherein the compound is represented by structural formula (II).
Embodiment 15. The method of embodiment 14, wherein M is linear C₂₋₄ alkylene, wherein the C₂₋₄ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 16. The method of embodiment 15, wherein the compound is represented by the following structural formula: 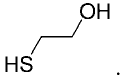
Embodiment 17. The method of embodiment 14, wherein M is branched C₃₋₅ alkylene, wherein the C₃₋₅ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 18. The method of embodiment 14, wherein the compound is represented by one of the following structural formulas: 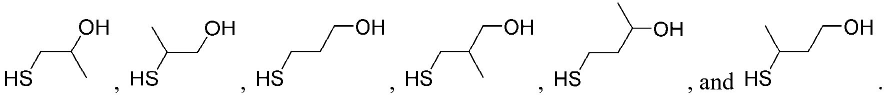
Embodiment 19. The method of any one of embodiments 1-18, wherein the thrombus comprises von Willebrand factor and platelet cells. In some embodiments, the concentration of von Willebrand factor is greater than about 0.1%, or greater than about 0.5%, or greater than about 1%, or greater than about 5%, or greater than about 10%, or greater than about 15%, or greater than about 20%, or greater than about 25%, or greater than about 30%, or greater than about 35%, or greater than about 40%, or greater than about 45%, or greater than about 50% of von Willebrand factor.
Embodiment 20. The method of embodiment 19, wherein the platelet cells are present at a concentration greater than about 5%. In some embodiments, the concentration of platelet cells is greater than about 0.1%, or greater than about 0.5%, or greater than about 1%, or greater than about 5%, or greater than about 10%, or greater than about 15%, or greater than about 20%, or greater than about 25%, or greater than about 30%, or greater than about 35%, or greater than about 40%, or greater than about 45%, or greater than about 50% of platelets.
Embodiment 21. The method of any one of embodiments 1-20, wherein the thrombus is substantially free of red blood cells. In some embodiments, the thrombus has a concentration of red blood cells of less than about 30%, or less than 25%, or less than 20% or less than 15% or less than 10%, or less than about 5%, or less than about 1%, or less than 0.5%.
Embodiment 22. The method of any one of embodiments 1-21, wherein the thrombus is substantially free of fibrin. In some embodiments, the fibrin has a concentration of less than about 30%, or less than 25%, or less than 20% or less than 15% or less than 10%, or less than about 5%, or less than about 1%, or less than 0.5%, or less than 0.1% or less than about 0.01%, or less than about 0.001%, or less than about 0.0001% of fibrin. However, in other embodiments, the thrombus has a substantial amount of fibrin, e.g., an amount greater than about 0.0001%, or greater than about 0.001%, or greater than about 0.01%, or greater than about 0.1%, or greater than about 1%, or greater than about 5%, or greater than about 10%, or greater than about 15% concentration of fibrin in the thrombus.
Embodiment 23. The method of any one of embodiments 1-22, wherein the thrombus formation occurs in a carotid artery of the subject.
Embodiment 24. The method of any one of embodiments 1-22, wherein the thrombus formation occurs in a coronary artery of the subject.
Embodiment 25. The method of any one of embodiments 1-24, wherein the treating results in a reduction of diameter of the thrombus by at least about 50%.
Embodiment 26. The method of embodiment 25, wherein the treating results in a reduction of diameter of the thrombus by at least about 70%.
Embodiment 27. The method of embodiment 26, wherein the treating results in a reduction of diameter of the thrombus by at least about 95%.
Embodiment 28. The method of any one of embodiments 1-27, wherein the compound is administered as a solution having a pH in the range of about 5 to about 8.
Embodiment 29. The method of embodiment 28, wherein the compound is administered as a solution having a pH of about 7.
Embodiment 30. The method of any one of embodiments 1-29, wherein the compound is administered as a solution at a concentration of about 0.1 mM to about 50 mM. In some embodiments, the compound is administered at a concentration range of about 0.01 mM to about 100 mM, or about 0.1 mM to about 90 mM, or about 1 mM to about 50 mM, or about 5 mM to about 20 mM. In some embodiments, the compound is administered at a concentration of about 1 mM, or about 2 mM, or about 3 mM, or about 4 mM, or about 5 mM, or about 6 mM, or about 7 mM, or about 8 mM, or about 9 mM, or about 10 mM, or about 11 mM, or about 12 mM, or about 13 mM, or about 14 mM, or about 15 mM, or about 16 mM, or about 17 mM, or about 18 mM, or about 19 mM, or about 20 mM concentration. In some embodiments, the compound is administered at a concentration of about 0.5 mM to about 50 mM. In some embodiments, the compound e is administered at a concentration of about 2 mM to about 20 mM. In some embodiments, the compound is administered at a concentration of about 3 mM to about 15 mM. In some embodiments, the compound is administered at a concentration of about 10 mM.
Embodiment 31. The method of embodiment 30, wherein the compound is administered as a solution at a concentration of about 0.5 mM to about 20 mM.
Embodiment 32. The method of embodiment 31, wherein the compound is administered as a solution at a concentration of about 1 mM to about 10 mM.
Embodiment 33. The method of embodiment 32, wherein the compound is administered as a solution at a concentration of about 10 mM.
Embodiment 34. The method of any one of embodiments 1-33, wherein the compound is administered in an amount from about 100 to about 1000 mg/kg.
Embodiment 35. The method of embodiment 34, wherein the compound is administered in an amount about 400 mg/kg.
Embodiment 36. The method of any one of embodiments 1-35, wherein the compound is administered once.
Embodiment 37. The method of any one of embodiments 1-35, wherein the compound is administered multiple times.
Embodiment 38. The method of any one of embodiments 1-37, wherein the subject is at high risk of forming arterial thrombi by having a blood concentration of von Willebrand factor higher than 50 IU/dL.
Embodiment 39. The method of any one of embodiments 1-38, wherein the subject is hospitalized for cardiovascular disease.
Embodiment 40. The method of any one of embodiments 1-39, wherein the subject is undergoing angioplasty or percutaneous coronary intervention, has a heart attack, or has ischemic stroke.
Embodiment 41. The method of any one of embodiments 1-40, wherein the compound is administered in combination with second agent.
Embodiment 42. The method of embodiment 41, wherein the second agent is a lytic agent.
Embodiment 43. The method of embodiment 42, wherein the lytic agent is selected from the group consisting of tissue plasminogen activator (tPA), recombinant tissue plasminogen activator (r-tPA), non-immunogenic recombinant streptokinase, recombinant human prourokinase, α-2-antiplasmin inhibitor, DNase-1, aptamer BB-03, aptamer BT200, aptamer DTRI-031, ADAMTS-13, dithiothreitol, N,N'-diacetyl-cystine (DiNAC), N-acetyl cysteine (NAC), and microlyse, or a combination thereof.
Embodiment 44. The method of embodiment 41, wherein the second agent is an anticoagulant agent.
Embodiment 45. The method of embodiment 44, wherein the anticoagulant agent is selected from the group consisting of warfarin, heparin or a derivative thereof, direct thrombin inhibitor, Factor XII inhibitor, Factor XI inhibitor, and Factor Xa inhibitor, or a combination thereof.
Embodiment 46. The method of embodiment 41, wherein the second agent is an antiplatelet agent.
Embodiment 47. The method of embodiment 46, wherein the antiplatelet agent is selected from the group consisting of aspirin, P2Y12 inhibitor, phosphodiesterase inhibitor, glycoprotein IIb/IIIa inhibitor, anti-GPVI antibody, and Protease-Activated Receptor-1 (PAR-1) antagonist, or a combination thereof.
Embodiment 48. The method of any one of embodiments 1-47, wherein the disease or disorder associated with thrombus formation is selected from the group consisting of arterial thrombosis, stroke, myocardial infraction, leg ischemia, a sickle-cell anemia, disseminated intravascular coagulation, extracorporeal circulation, heart failure, valvular disease, aortic stenosis, and venous thrombosis.
Embodiment 49. The method of any one of embodiments 1-48, wherein the compound is administered as a liquid dosage form.
Embodiment 50. The method of any one of embodiments 1-49, wherein the compound is administered to the subject intravenously.
Embodiment 51. The method of any one of embodiments 1-48, wherein the compound is administered as an oral dosage form.
Embodiment 52. The method of embodiment 51, wherein the oral dosage form is a liquid. Embodiment 53. The method of any one of embodiments 1-52, wherein the subject is a human subject.

In a 54^{th} embodiment the present disclosure relates to a method of treating thrombus formation in a cavity or device, comprising contacting the cavity or device with a compound represented by structural formula (I) or structural formula (II) or a pharmaceutically acceptable salt thereof:

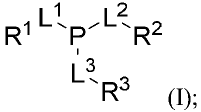

HO-M-SH (II),

wherein
each L¹, L², and L³ is independently C₁₋₆ alkylene;
M is C₂₋₆ alkylene;
each R¹, R², and R³ is independently selected from C(O)OR⁵, OR⁴, C(O)NR⁶R⁷, NR⁸C(O)R⁹, and NR¹⁰R¹¹,
each R⁴, R⁵, R⁶, R⁷, R⁸, R⁹, R¹⁰, and R¹¹ is selected from H and C₁₋₆ alkyl, or
R⁶ and R⁷, or R¹⁰ and R¹¹, together with the nitrogen atom to which they are attached form a 3- to 8-membered heterocyclyl,
wherein each C₁₋₆ alkylene, C₂₋₆ alkylene, and C₁₋₆ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{21b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
wherein
each R¹¹, R¹², R¹³, R¹⁴, R^{14*}, R¹⁵, R^{15*}, R¹⁶, R¹⁷, R^{16*}, R^{17*}, R¹⁸, R^{18a}, R¹⁹, R²⁰, R^{20a}, R^{20b}, R²¹, R²², R^{21*}, R^{22*}, R²³, R²⁴, R²⁵, R²⁶, and R²⁷ is independently selected from H, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl, or
one or more of the pairs of variables selected from R¹² and R¹³, R¹⁶ and R¹⁷, R^{16*} and R^{17*}, R²¹ and R²², R^{21*} and R^{22*}, and R²⁵ and R²⁶, together with the nitrogen to which they are attached, form a 3-8 membered ring, and
each of the substituents selected from C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl is further optionally substituted with 1 to 3 substituents independently selected from deuterium, oxo, F, Cl, Br, CN, NO₂, OR^{14*}, SR^{15*}, NR^{16*}R^{17*}, C₁₋₆ alkyl, C₁₋₆ haloalkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.

Embodiment 55. The method of embodiment 54, wherein the compound is represented by structural formula (I).
Embodiment 56. The method of embodiment 54 or 55, wherein L¹, L², and L³ are each C₂₋₃ alkylene, wherein the C₂₋₃ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 57. The method of any one of embodiments 54-56, wherein each R¹, R², and R³ is independently selected from C(O)NR⁶R⁷, NR⁸C(O)R⁹, and NR¹⁰R¹¹.
Embodiment 58. The method of any one of embodiments 54-56, wherein the compound is represented by structural formula (Ia): 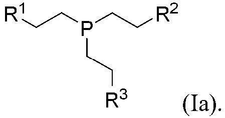
Embodiment 59. The method of embodiment 58, wherein the compound is represented by structural formula (Ib): 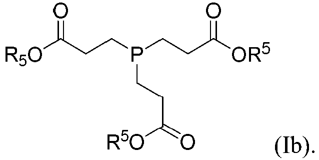
Embodiment 60. The method of embodiment 59, wherein each R⁵ is independently selected from H and C₁₋₃ alkyl, wherein each C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 61. The method of embodiment 60, wherein the compound is represented by one of the following structural formulas: 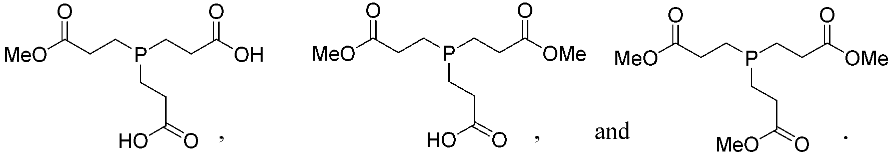
Embodiment 62. The method of embodiment 60, wherein the compound is represented by the following structural formula: 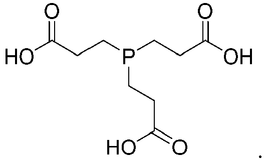
Embodiment 63. The method of any one of embodiments 54-56, wherein the compound is represented by structural formula (Ic): 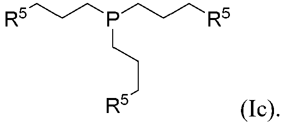
Embodiment 64. The method of embodiment 63, wherein the compound is represented by structural formula (Id): 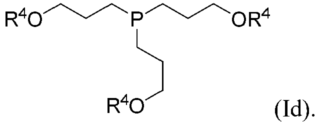
Embodiment 65. The method of embodiment 64, wherein each R⁴ is independently selected from H and C₁₋₃ alkyl, wherein the C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 66. The method of embodiment 65, wherein the compound is represented by the following structural formula: 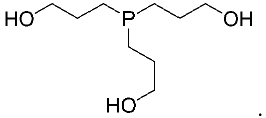
Embodiment 67. The method of embodiment 54, wherein the compound is represented by structural formula (II).
Embodiment 68. The method of embodiment 67, wherein M is linear C₂₋₄ alkylene, wherein the C₂₋₄ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 69. The method of embodiment 68, wherein the compound is represented by the following structural formula: 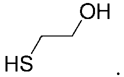
Embodiment 70. The method of embodiment 67, wherein M is branched C₃₋₅ alkylene, wherein the C₃₋₅ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl.
Embodiment 71. The method of embodiment 67, wherein the compound is represented by one of the following structural formulas: 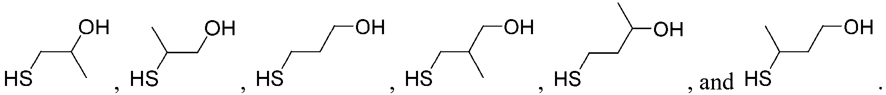
Embodiment 72. The method of any one of embodiments 55-71, wherein the cavity or device comprises tubing, a valve, a graft, a circuit, a stent, a catheter, or a thrombectomy device.
Embodiment 73. The method of embodiment 72, wherein the tubing is a blood tubing.
Embodiment 74. The method of embodiment 72, wherein the valve is a heart valve.
Embodiment 75. The method of embodiment 72, wherein the graft is a dialysis graft.
Embodiment 76. The method of any one of embodiments 54-75, wherein the thrombus comprises von Willebrand factor and platelet cells.
Embodiment 77. The method of embodiment 76, wherein the platelet cells are present at a concentration greater than about 5%.
Embodiment 78. The method of any one of embodiments 54-77, wherein the thrombus is substantially free of red blood cells.
Embodiment 79. The method of any one of embodiments 54-78, wherein the thrombus is substantially free of fibrin.
Embodiment 80. The method of any one of embodiments 54-79, wherein the treating results in a reduction of diameter of the thrombus by at least about 50%.
Embodiment 81. The method of embodiment 80, wherein the treating results in a reduction of diameter of the thrombus by at least about 70%.
Embodiment 82. The method of embodiment 81, wherein the treating results in a reduction of diameter of the thrombus by at least about 95%.
Embodiment 83. The method of any one of embodiments 54-82, wherein the compound is administered as a solution having a pH in the range of about 5 to about 8.
Embodiment 84. The method of embodiment 83, wherein the compound is administered as a solution having a pH of about 7.
Embodiment 85. The method of any one of embodiments 54-84, wherein the compound is administered as a solution at a concentration of about 0.1 mM to about 50 mM.
Embodiment 86. The method of embodiment 85, wherein the compound is administered as a solution at a concentration of about 0.5 mM to about 20 mM.
Embodiment 87. The method of embodiment 86, wherein the compound is administered as a solution at a concentration of about 1 mM to about 10 mM.
Embodiment 88. The method of embodiment 87, wherein the compound is administered as a solution at a concentration of about 10 mM.
Embodiment 89. The method of any one of embodiments 54-88, wherein the compound is administered in combination with a second agent.
Embodiment 90. The method of embodiment 89, wherein the second agent is a lytic agent.
Embodiment 91. The method of embodiment 90, wherein the lytic agent is selected from the group consisting of tissue plasminogen activator (tPA), recombinant tissue plasminogen activator (r-tPA), non-immunogenic recombinant streptokinase, recombinant human prourokinase, α-2-antiplasmin inhibitor, DNase-1, aptamer BB-03, aptamer BT200, aptamer DTRI-031, ADAMTS-13, dithiothreitol, N,N'-diacetyl-cystine (DiNAC), N-acetyl cysteine (NAC), and microlyse, or a combination thereof.
Embodiment 92. The method of embodiment 89, wherein the second agent is an anticoagulant agent.
Embodiment 93. The method of embodiment 92, wherein the anticoagulant agent is selected from the group consisting of warfarin, heparin or a derivative thereof, direct thrombin inhibitor, Factor XII inhibitor, Factor XI inhibitor, and Factor Xa inhibitor, or a combination thereof.
Embodiment 94. The method of embodiment 89, wherein the second agent is an antiplatelet agent.
Embodiment 95. The method of embodiment 94, wherein the antiplatelet agent is selected from the group consisting of aspirin, P2Y12 inhibitor, phosphodiesterase inhibitor, glycoprotein IIb/IIIa inhibitor, anti-GPVI antibody, and Protease-Activated Receptor-1 (PAR-1) antagonist, or a combination thereof.
Embodiment 96. The method of any one of embodiments 1-53, wherein the compound represented by structural formula (I) treats the disease or disorder.
Embodiment 97. The method of any one of embodiments 54-95, wherein the compound represented by structural formula (I) treats the thrombus formation.

### Exemplification

### Example 1. Design, Materials, and Methods

### Experimental Design

Thrombi were formed from human heparinized whole blood in a microfluidic system with high shear rate over a surface coated with fibrillar collagen. After occlusion, the thrombus was perfused with a solution containing a lytic agent. The lytic agents used PBS or whole human blood as the solvent. PBS assessed intrinsic lytic potential with minimal blood use, while blood captured lysis with any complex physiological interactions with whole blood.

### Whole human blood

Blood was obtained from consenting, healthy volunteers following approval from the Georgia Tech Institutional Review Board #17315. All participants were over 18 years old and had not taken antiplatelet medications for at least 10 days before the blood collection. Individuals with known anemia, transmissible blood diseases, or bleeding disorders were excluded in the study. Blood was drawn by a professional phlebotomist into a 60 mL syringe containing low dose heparin (3.5 IU/mL) to prevent blood clotting during transport.

### Thrombolysis assay

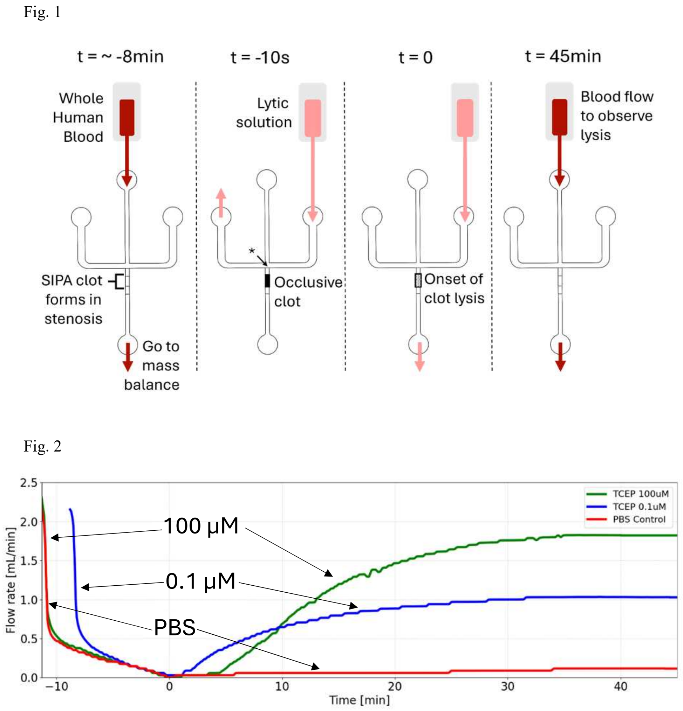

The microfluidic mold was manufactured using a 3D printer (Elegoo Saturn 2). The microfluidic PDMS chip featured a trifurcation channel upstream to a stenotic test section. The trifurcation allowed the transportation of lytic agents to occlusive clots within seconds. The dimensions of the stenotic test section were 200*400*1000 µm. SIPA thrombi were formed in the stenotic test section by perfusing gra heparin concentration (3.5 IU/mL) (Fig. 1). The chamber was coated overnight with 100 µg/mL equine collagen fibrils type I (Chrono-Log Corp) to render the glass slide surface thrombogenic. The flow was driven by a first pressure column (~20 mmHg) containing blood inducing a high initial wall shear rate in the stenotic region (~5000/s). A second pressure column (~20 mmHg) was used to drive the flow of the lytic solution containing the disulfide reducing agent. PBS was used as vehicle for experiments with TCEP, THPP, and BME in the lytic solution and drops of blood were used to color the solution for visualization. Whole human blood was used as vehicle for experiments with TCEP. A mass balance measured the outflow mass over time. A microscope focused on the microfluidic channel was used to record images of the lysis experiment. Prior to perfusing with blood, the tubing and the microfluidic channel were primed with PBS. The experiment was started by opening the valve of the first pressure column to begin flowing blood. The thrombus started to form when blood reached the microfluidic channel. Occlusion was considered reached when the measured flow rate was lower than 5% of its initial value. When this criterion was met, the valve of the first pressure column containing blood was closed. Then, the valve of the second pressure column was open to begin flowing the lytic solution. To improve convection of the lytic agent to the trifurcation, the outlet of the trifurcation channel was opened for a few seconds to flush away the PBS between the thrombus and the second pressure column. Light microscopy confirmed that PBS was flushed away and that the lytic solution properly reached the thrombus. When the lytic agent reached the thrombi, the trifurcation channel was closed, marking the start of the lysis part of the experiment. Thrombolysis was observed with light microscopy and quantified with a mass balance for 45 minutes from the onset of the lysis part.

### Compounds evaluated for thrombolytic efficacy

The thrombolytic efficacy of three disulfide bond reducing agents was evaluated on platelet and VWF rich thrombi formed under high shear hemodynamics conditions: Tris(2-carboxyethyl)phosphine (TCEP) (Thermo Scientific, USA), Tris(hydroxypropyl)phosphine (THPP) (MilliporeSigma, USA), and 2-mercaptoethanol (BME) (TCI Chemicals, Japan). The tested concentrations ranged from 0.1µM to 10mM for TCEP and 100µM to 10mM for BME. THPP was tested at a 5mM concentration. For TCEP, THPP, and BME, PBS was used as vehicle in the lytic solution and drops of blood equivalent to 10% of the final volume of the lytic solution were added to color it. In addition, TCEP was also tested using whole human blood as a vehicle in the lytic solution (90% blood and 10% PBS).

### Flow Rate Assay

The flow rate in the stenosis was calculated by numerical differentiation of mass values using a 1 second step size. The flow rate was measured from the onset of the experiment when the blood in the pressure head starts flowing through the tubes, to the end of the experiment when the lytic agent has been perfused for 45 minutes. Percent lytic efficacy was defined as the ratio of the final flow rate (45 minutes after perfusion of the lytic agent) to the initial flow rate (prior to blood perfusion for thrombus formation), multiplied by 100. TCEP efficacy cannot reach 100% when PBS is the vehicle because the final flow rate is calculated with a more viscous solution than the initial flow rate (initial flow 100% blood vs. final flow 10% blood and 90% PBS).

### Tail Bleeding Assay

The *in vivo* bleeding profile of TCEP was tested using a murine tail vein transection model adapted from Illa et al., Tail Vein Transection Bleeding Model in Fully Anesthetized Hemophilia A Mice. J. Vis. Exp. (175), e62952, doi:10.3791/62952 (2021). Adult healthy C57BL/6 mice were fully anesthetized (isoflurane induction 5%, maintenance 2%) throughout the terminal experiment. The right jugular vein was exposed and 200 µL of saline or saline containing heparin 200 IU or TCEP 90 mg/kg was administered intravenously using an insulin syringe. The incision was closed within 2 min. The tail was pre-warmed for 5 min in 37 °C saline. Seven minutes after injection, the left lateral tail vein was transected using a standardized template guide, and the tail was immediately immersed in saline. Bleeding episodes were continuously monitored for 40 min. If no bleeding occurred at 10, 20, or 30 min post-injury, the clot was challenged by gently disrupting by wiping the wound twice with a wet gauze swab. Occlusion was defined as the absence of bleeding for >30 s. The results of the Tail Bleeding Assay are shown below.

### Example 2. Lytic potential data for TCEP, BME, and THPP

In the study described herein, human whole blood with low heparin concentration (3.5 IU/mL) was perfused through a stenotic chamber coated with fibrillar collagen (shear rate >6,000/s) until a SIPA thrombus was formed (Fig. 1). Occlusion was determined by flow rate measurement derived from mass data. The occlusive high shear thrombus observed through light microscopy was then perfused with a lytic solution. The recanalization of the stenotic chamber was quantified by flow rate. Three flow rate curves from blood flowing onset to 45 minutes post lysis onset were compared as an example (Fig. 2). While TCEP 100 µM recanalized the microfluidic channel within 30 minutes, TCEP 0.1 µM achieved partial recanalization and the control test using PBS only did not achieve recanalization.

Light microscopy comparison images show the difference in degree of recanalization of the occluded microfluidic channel (Fig. 3). 45 minutes following the perfusion of lytic solution of TCEP and PBS as vehicle, SIPA material was partially (Fig. 3A) or completely dissolved (Fig. 3B), while control test with PBS only showed no dissolution (Fig. 3C). Interestingly, flow rate with TCEP 0.1 µM plateaued (Fig. 2) at 50% the value of the final flow rate seen with TCEP 100 µM, and light microscopy images of TCEP 0.1 µM show the presence of small white patches in the channel after 45 minutes (Fig. 3A). The decrease in pressure and shear forces on occlusive thrombus due to recanalization may also allow the clot not to fully dissolve as with TCEP 0.1 µM (Fig. 3A).

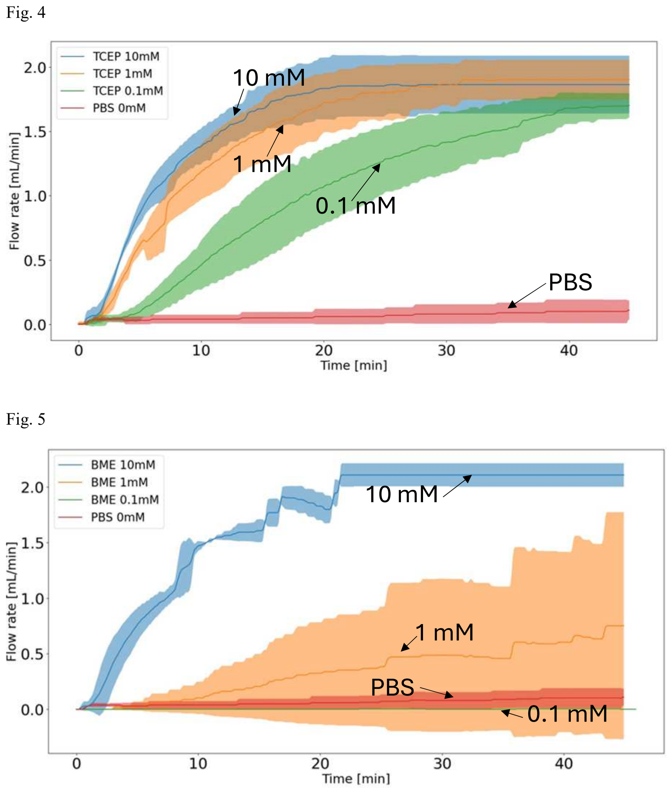

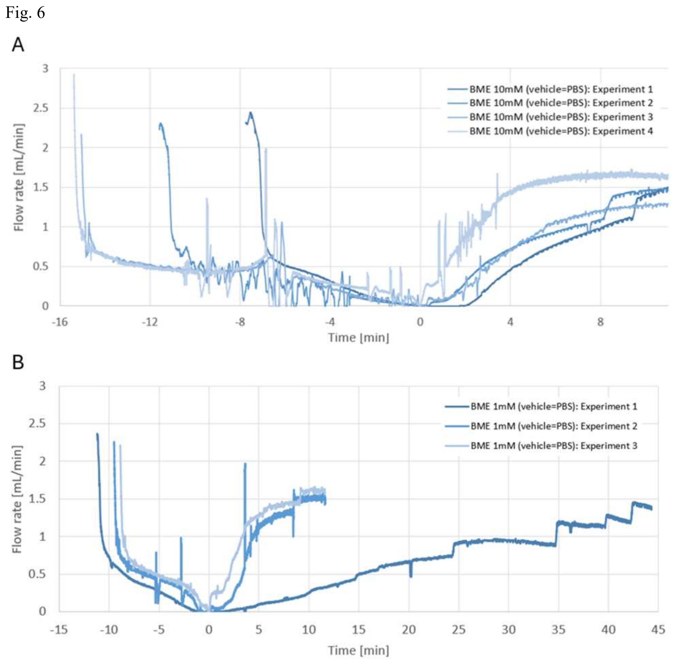

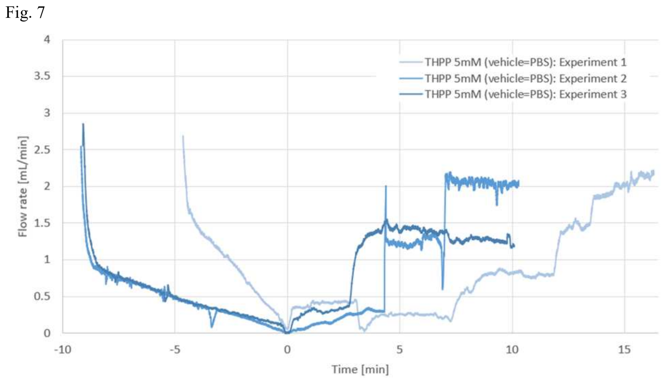

The lytic potential of BME and THPP, two other disulfide bond reducing agents, was tested on high shear thrombi (Figs. 5-7). TCEP 100 µM (experiment performed 4 times) proved to have a slower but as efficacious recanalization profile as TCEP 10 mM (experiment performed 3 times) and TCEP 1 mM (experiment performed 4 times) after 45 minutes (Fig. 4). In a first batch of experiments, BME 10 mM recanalized the stenotic chamber (experiment performed 2 times) (Fig. 5). BME 1 mM recanalized the stenotic chamber in one out of two experiments, explaining the large standard deviation observed in the figure (Fig. 5). BME 0.1mM did not recanalize (experiment performed 2 times) (Fig. 5). To increase the number of replicates, a second batch of experiments was conducted in which BME 10 mM (experiment performed 4 times) (Fig. 6 (panel A)) and BME 1mM (experiment performed 3 times) (Fig. 6 (panel B)) recanalized the stenotic chamber. In one BME 1 mM replicate, recanalization was slower, reaching same flow level at 45 minutes that the two others BME 1mM reached at 10 minutes. THPP 5 mM also recanalized the stenotic chamber (experiment performed 3 times) (Fig. 7) . TCEP, BME, and THPP managed to dissolve the bulk of thrombi at millimolar concentrations levels. These results indicate that disulfide bonds play a significant role in maintaining the structural stability of high-shear thrombi.

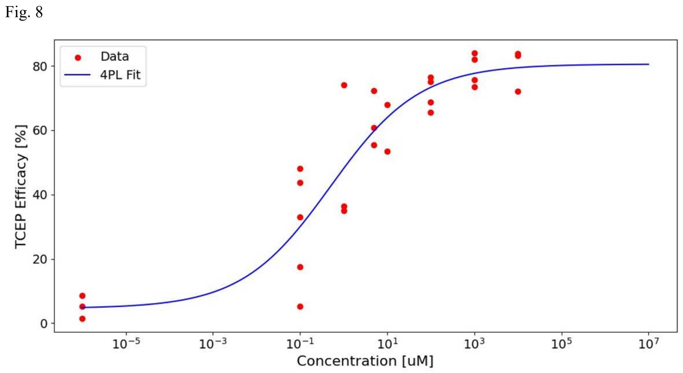

The thrombolytic potential of TCEP was explored at lower concentrations (Fig. 8). Concentrations as low as 0.1 µM partially recanalized the microfluidic channel (Figs. 3A & 8), averaging a recanalization level of 30%. Concentrations higher than 5 µM managed to recanalize at more than 60% from maximum possible flow.

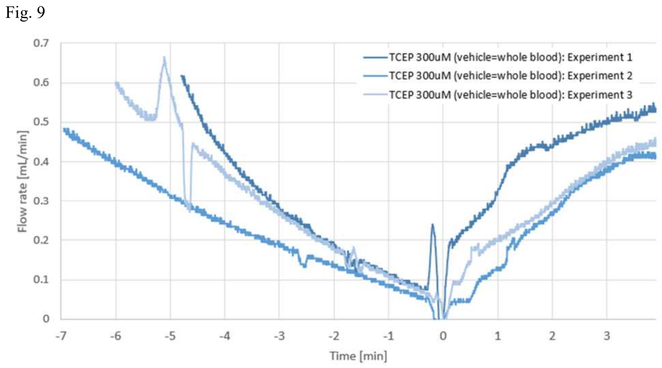

The lytic potential of TCEP in whole human blood was tested to assess whether TCEP still dissolves clots even in an environment that promotes thrombi formation. TCEP 300 µM (experiment performed 3 times) with blood as vehicle recanalized the stenotic chamber to initial flow levels within minutes (Fig. 9). This result demonstrates the efficacy of TCEP at lysing high shear thrombi in an arterial environment.

### Tail Bleeding Assay Results

The bleeding safety profile of TCEP was assessed using the Tail Bleeding Assay described above. Results are shown in Fig. 10, which illustrates how bleeding occurred for each individual mouse in the saline (control), TCEP, and heparin groups. The tail started bleeding at the 0-minute timepoint following the tail vein transection. The length of the first bleeding episode in the heparin (198 ± 65 s) and TCEP (223 ± 79 s) groups was on average two-times longer than in the saline group (97 ± 23 s), suggesting that heparin and TCEP mildly attenuate primary hemostasis without preventing the formation of an occlusive clot within minutes. No bleeding occurred at the 10-minute timepoint in all groups; the occlusive clot was then challenged by wiping the cut, which resulted in a second bleeding episode. While the saline (122 ± 32 s) and TCEP (105 ± 19 s) groups stopped bleeding within approximately 2 minutes, the heparin (1,545 ± 355 s) group bled ten times longer, for 20-plus minutes. Challenges were repeated at 20 and 30 minutes in the saline and TCEP groups resulting in a third then a fourth bleeding episode. Both groups had similar bleeding times (TCEP: 86 ± 19 s; 82 ± 37 s) (saline: 94 ± 15 s; 105 ± 10 s). These results indicate that TCEP did not impair secondary hemostasis.

While this invention has been particularly shown and described with references to example embodiments thereof, it will be understood by those skilled in the art that various changes in form and details may be made therein without departing from the scope of the invention encompassed by the appended claims.

## Claims

1. A compound represented by structural formula (I) or structural formula (II) or a pharmaceutically acceptable salt thereof: 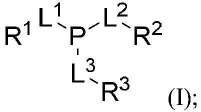
HO-M-SH (II),
wherein
each L¹, L², and L³ is independently C₁₋₆ alkylene;
M is C₂₋₆ alkylene;
each R¹, R², and R³ is independently selected from C(O)OR⁵, OR⁴, C(O)NR⁶R⁷, NR⁸C(O)R⁹, and NR¹⁰R¹¹,
each R⁴, R⁵, R⁶, R⁷, R⁸, R⁹, R¹⁰, and R¹¹ is selected from H and C₁₋₆ alkyl, or
R⁶ and R⁷, or R¹⁰ and R¹¹, together with the nitrogen atom to which they are attached form a 3- to 8-membered heterocyclyl,
wherein each C₁₋₆ alkylene, C₂₋₆ alkylene, and C₁₋₆ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
wherein
each R¹¹, R¹², R¹³, R¹⁴, R^{14*}, R¹⁵, R^{15*}, R¹⁶, R¹⁷, R^{16*}, R^{17*}, R¹⁸, R^{18a}, R¹⁹, R²⁰, R^{20a}, R^{20b}, R²¹, R²², R^{21*}, R^{22*}, R²³, R²⁴, R²⁵, R²⁶, and R²⁷ is independently selected from H, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl, or
one or more of the pairs of variables selected from R¹² and R¹³, R¹⁶ and R¹⁷, R^{16*} and R^{17*}, R²¹ and R²², R^{21*} and R^{22*}, and R²⁵ and R²⁶, together with the nitrogen to which they are attached, form a 3-8 membered ring, and
each of the substituents selected from C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl is further optionally substituted with 1 to 3 substituents independently selected from deuterium, oxo, F, Cl, Br, CN, NO₂, OR^{14*}, SR^{15*}, NR^{16*}R^{17*}, C₁₋₆ alkyl, C₁₋₆ haloalkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl,
for use in a method of treating a disease or disorder associated with thrombus formation in a subject in need thereof, comprising administering to the subject a therapeutically effective amount of said compound or pharmaceutically acceptable salt thereof.

2. The compound for use according to claim 1, wherein the compound is represented by structural formula (I), optionally wherein:
(a) L¹, L², and L³ are each C₂₋₃ alkylene, wherein the C₂₋₃ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl; and/or
(b) each R¹, R², and R³ is independently selected from C(O)NR⁶R⁷, NR⁸C(O)R⁹, and NR¹⁰R¹¹.

3. The compound for use according to claim 1 or claim 2, wherein the compound is represented by:
(a) structural formula (Ia) 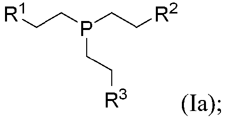
(b) structural formula (Ib) 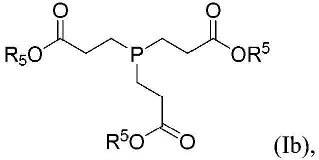 optionally wherein each R⁵ is independently selected from H and C₁₋₃ alkyl, wherein each C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl;
(c) one of the following structural formulas 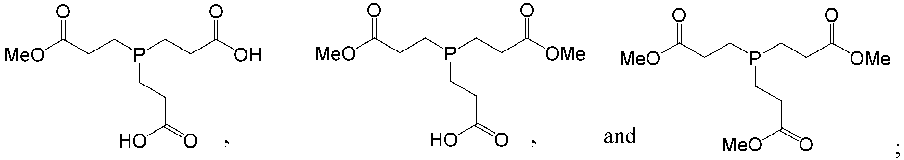
(d) the following structural formula 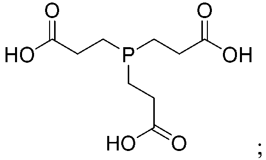
(e) structural formula (Ic) 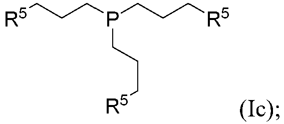
(f) structural formula (Id) 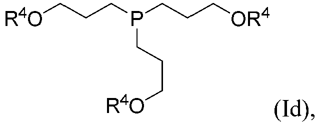 optionally wherein each R⁴ is independently selected from H and C₁₋₃ alkyl, wherein the C₁₋₃ alkyl is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5-to 12-membered heteroaryl; or
(g) the following structural formula

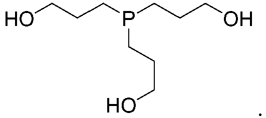

4. The compound for use according to claim 1, wherein the compound is represented by structural formula (II).

5. The compound for use according to claim 4, wherein:
(a) M is linear C₂₋₄ alkylene, wherein the C₂₋₄ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl;
(b) the compound is represented by the following structural formula 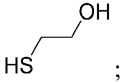
(c) M is branched C₃₋₅ alkylene, wherein the C₃₋₅ alkylene is optionally independently substituted with 1 to 3 substituents selected from deuterium, oxo, F, Cl, Br, CN, NO₂, OR¹⁴, SR¹⁵, NR¹⁶R¹⁷, S(O)R¹⁸, S(O)₂R^{18a}, NR¹⁹S(=O)R²⁰, C(=O)OR^{20a}, OC(=O)OR^{20b}, C(=O)NR²¹R²², OC(=O)NR^{21*}R^{22*}, NR¹¹C(=O)N(R¹²)(R¹³), NR²³C(=O)R²⁴, C(=S)NR²⁵R²⁶, C(=O)R²⁷, C₁₋₆ alkyl, C₂₋₆ alkenyl, C₂₋₆ alkynyl, C₃₋₁₂ cycloalkyl, C₆₋₁₂ aryl, 4- to 12-membered heterocyclyl, and 5- to 12-membered heteroaryl; or
(d) the compound is represented by one of the following structural formulas:

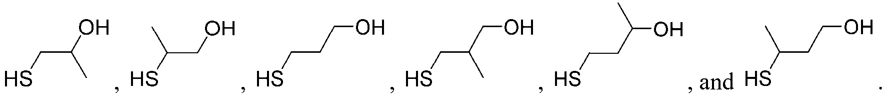

6. The compound for use according to any one of claims 1-5, wherein:
(a) the thrombus comprises von Willebrand factor and platelet cells, optionally wherein the platelet cells are present at a concentration greater than about 5%;
(b) the thrombus is substantially free of red blood cells;
(c) the thrombus is substantially free of fibrin;
(d) the thrombus formation occurs in a carotid artery of the subject, or in a coronary artery of the subject;
(e) the treating results in a reduction of diameter of the thrombus by at least about 50%, such as by at least about 70% or by at least about 95%;
(f) the compound is administered as a solution having a pH in the range of about 5 to about 8, such as a pH of about 7;
(g) the compound is administered as a solution at a concentration of about 0.1 mM to about 50 mM, such as about 0.5 mM to about 20 mM about 1 mM to about 10 mM or about 10 mM; and/or
(h) the compound is administered in an amount from about 100 to about 1000 mg/kg, such as about 400 mg/kg.

7. The compound for use according to any one of claims 1-6, wherein the compound is administered once.

8. The compound for use according to any one of claims 1-6, wherein the compound is administered multiple times.

9. The compound for use according to any one of claims 1-8, wherein the subject:
(a) is at high risk of forming arterial thrombi by having a blood concentration of von Willebrand factor higher than 50 IU/dL;
(b) is hospitalized for cardiovascular disease; and/or
(c) is undergoing angioplasty or percutaneous coronary intervention, has a heart attack, or has ischemic stroke.

10. The compound for use according to any one of claims 1-9, wherein the compound is administered in combination with second agent, optionally wherein the second agent is:
(a) a lytic agent, such as a lytic agent selected from the group consisting of tissue plasminogen activator (tPA), recombinant tissue plasminogen activator (r-tPA), non-immunogenic recombinant streptokinase, recombinant human prourokinase, α-2-antiplasmin inhibitor, DNase-1, aptamer BB-03, aptamer BT200, aptamer DTRI-031, ADAMTS-13, dithiothreitol, N,N'-diacetyl-cystine (DiNAC), N-acetyl cysteine (NAC), and microlyse, or a combination thereof;
(b) an anticoagulant agent, such as an anticoagulant agent selected from the group consisting of warfarin, heparin or a derivative thereof, direct thrombin inhibitor, Factor XII inhibitor, Factor XI inhibitor, and Factor Xa inhibitor, or a combination thereof; or
(c) an antiplatelet agent, such as an antiplatelet agent selected from the group consisting of aspirin, P2Y12 inhibitor, phosphodiesterase inhibitor, glycoprotein IIb/IIIa inhibitor, anti-GPVI antibody, and Protease-Activated Receptor-1 (PAR-1) antagonist, or a combination thereof.

11. The compound for use according to any one of claims 1-10, wherein:
(a) the disease or disorder associated with thrombus formation is selected from the group consisting of arterial thrombosis, stroke, myocardial infraction, leg ischemia, a sickle-cell anemia, disseminated intravascular coagulation, extracorporeal circulation, heart failure, valvular disease, aortic stenosis, and venous thrombosis; and/or
(b) the subject is a human subject.

12. The compound for use according to any one of claims 1-11, wherein:
(a) the compound is administered as a liquid dosage form and/or the compound is administered to the subject intravenously; or
(b) the compound is administered as an oral dosage form, optionally wherein the oral dosage form is a liquid.

13. An *ex vivo* method of treating thrombus formation in a cavity or device, comprising contacting the cavity or device with a compound represented by structural formula (I) or structural formula (II) or a pharmaceutically acceptable salt thereof, wherein
the compound represented by structural formula (I) is as defined in any one of claims 1 to 3; and the compound represented by structural formula (II) is as defined in any one of claims 1, 4 and 5.

14. The *ex vivo* method of claim 12, wherein the cavity or device comprises tubing, a valve, a graft, a circuit, a stent, a catheter, or a thrombectomy device, optionally wherein:
(a) the tubing is a blood tubing;
(b) the valve is a heart valve; or
(c) the graft is a dialysis graft.

15. The *ex vivo* method of claims 13 or claim 14, wherein:
(a) the thrombus comprises von Willebrand factor and platelet cells, optionally wherein the platelet cells are present at a concentration greater than about 5%;
(b) thrombus is substantially free of red blood cells;
(c) the thrombus is substantially free of fibrin;
(d) the treating results in a reduction of diameter of the thrombus by at least about 50%, such as by at least about 70% or by at least about 95%;
(e) the compound is administered as a solution having a pH in the range of about 5 to about 8, such as about 7;
(f) the compound is administered as a solution at a concentration of about 0.1 mM to about 50 mM, such as about 0.5 mM to about 20 mM, about 1 mM to about 10 mM or about 10 mM;
(g) the compound is administered in combination with a second agent, wherein the second agent is as defined in claim 10.
